(19) Europäisches Patentamt
European Patent Office
Office européen des brevets

(11) EP 4 203 948 B1

(12) EUROPEAN PATENT SPECIFICATION

(45) Date of publication and mention of the grant of the patent:
**30.09.2026 Bulletin 2026/40**

(21) Application number: **21799200.7**

(22) Date of filing: **15.10.2021**

(51) International Patent Classification (IPC):
***A61K 31/405*** *(2006.01)* ***A61K 31/404*** *(2006.01)*
***A61K 31/48*** *(2006.01)* ***A61P 25/00*** *(2006.01)*
***A61K 31/4045*** *(2006.01)* ***A61K 31/15*** *(2006.01)*

(52) Cooperative Patent Classification (CPC):
(C-Sets available)
**A61K 31/4045; A61K 31/15; A61K 31/405; A61K 31/48; A61K 31/4985; A61P 25/00** (Cont.)

(86) International application number:
**PCT/EP2021/078716**

(87) International publication number:
**WO 2022/079304 (21.04.2022 Gazette 2022/16)**

# (54) COMPOSITION COMPRISING TRYPTOPHANS FOR USE IN THE TREATMENT OF FMR1-MEDIATED DISORDERS

ZUSAMMENSETZUNG, DIE TRYPTOPHANE ENTHÄLT, ZUR VERWENDUNG BEI DER BEHANDLUNG VON DURCH FMR1 VERMITTELTEN ERKRANKUNGEN

COMPOSITION COMPRENANT DES TRYPTOPHANES POUR UTILISATION DANS LE TRAITEMENT DE TROUBLES MÉDIÉS PAR FMR1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2020 GB 202016492**
**05.08.2021 GB 202111344**

(43) Date of publication of application:
**05.07.2023 Bulletin 2023/27**

(73) Proprietor: **Purposeful A.E.**
**11251 Athens (GR)**

(72) Inventors:
- **DRAKAKIS, Georgios**
  **11251 Athens (GR)**
- **CHOMENIDIS, Charalampos**
  **11251 Athens (GR)**
- **TSILIKI, Georgia**
  **11251 Athens (GR)**

(74) Representative: **Appleyard Lees IP LLP**
**G Mill**
**Dean Clough Industrial Park**
**Halifax HX3 5AH (GB)**

(56) References cited:
**WO-A1-2020/011753** **US-A- 5 885 976**
**US-A1- 2006 142 375** **US-B2- 9 468 627**

- **JACOBSEN JACOB P R ET AL: "Adjunctive 5-Hydroxytryptophan Slow-Release for Treatment-Resistant Depression: Clinical and Preclinical Rationale", TRENDS IN PHARMACOLOGICAL SCIENCES, ELSEVIER, HAYWARTH, GB, vol. 37, no. 11, 28 September 2016 (2016-09-28), pages 933 - 944, XP029774474, ISSN: 0165-6147, DOI: 10.1016/J.TIPS.2016.09.001**
- **PROTIC DRAGANA ET AL: "New Targeted Treatments for Fragile X Syndrome", CURRENT PEDIATRIC REVIEWS, vol. 15, no. 4, 30 December 2019 (2019-12-30), pages 251 - 258, XP055825497, ISSN: 1573-3963, DOI: 10.2174/1573396315666190625110748**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 203 948 B1

(52) Cooperative Patent Classification (CPC): (Cont.)

C-Sets
**A61K 31/15, A61K 2300/00;**
**A61K 31/4045, A61K 2300/00;**
**A61K 31/405, A61K 2300/00;**
**A61K 31/48, A61K 2300/00;**
**A61K 31/4985, A61K 2300/00**

## Description

Technical Field of the Invention

The invention relates to compositions for use in the treatment, management or amelioration of neurological and developmental disorders, and in particular for the treatment of a range of autism diseases or diseases where autism is a known component treatment of and also Fragile X Syndrome (FXS).

Background to the Invention

Mutational inactivation of the gene encoding the Fragile X Mental Retardation protein (FMRP) causes a spectrum of symptoms including seizures, sleep disorders, anxiety, irritability, autism, mild to severe cognitive impairment and intellectual disability. The constellation of symptoms is known as Fragile-X syndrome (FXS).

FXS is caused by the transcriptional silencing of the FMR1 gene (Xq27.3) due to the progressive expansion and subsequent methylation of (CGG)n trinuleotide repeats in the 5'-untranslated region of the gene. These full mutations originate from unstable alleles called premutations (55-200 CGG repeats). In some rare cases, FXS was shown to result from intragenic FMR1 point mutations or deletions. FMR1 codes for the FMRP, an RNA-binding protein that regulates protein synthesis and other signaling pathways in neuronal dendrites. FMR1 silencing is thought to reduce synaptic plasticity and modulation throughout the brain including the hippocampus.

The syndrome in humans is caused by expansion of an unstable, CGG triplet expansion (> 200 repeats) in the 5' untranslated region of the Fmr1 gene located on the X chromosome, which leads to gene methylation, inactivation, and resultant loss of fragile X mental retardation protein expression (FMRP). FMRP functions as a translational regulator, affecting synthesis of many proteins including those involved in synaptic pruning during development (Razak, 2020). Meta-analysis estimates the frequencies of individuals with the full mutation FXS allele to be approximately 1 in 7000 males and 1 in 11,000 females (Hunter, 2014). FXS is severely debilitating in males. Females generally are less affected than males due to mosaicism resulting from X-chromosome inactivation which occurs randomly early in embryogenesis (ME Gurney, 2017).

Fragile X syndrome (FXS) presents with a variable clinical phenotype. In males, the disease presents during childhood with delayed developmental milestones. Intellectual deficit can be of variable severity and may include problems with working and short-term memory, executive function, language, mathematics and visuospatial abilities. Behavioral anomalies can be mild (e.g. anxiety, mood instability) to severe (e.g. aggressive behavior, autism). Autistic-like behavior can include hand flapping, poor eye contact, hand biting, gaze avoidance, social phobia, social and communication deficits and tactile defensiveness. In females, intellectual and behavioral disorders are typically mild and usually consist of shyness, social anxiety, and mild learning problems with a normal IQ, although 25% of girls have an IQ less than 70. Attention deficit hyperactivity disorder (ADHD) is present in over 89% of males and 30% of females and behavioral disinhibition is very common. Recurrent otitis (60%) and seizures (16 to 20%) can also be observed. FXS patients display a range of neuropsychiatric symptoms including intellectual disability, delayed language acquisition, poor social interaction, hyperarousal, hypersensitivity, repetitive behaviors, disrupted sleep, attention deficit hyperactivity disorder (ADHD) and autism. These behavioral changes are most widely modelled in adult male Fmr1 knockout (KO) mice which display a spectrum of behavioral phenotypes due to the fmr1 gene deletion. The mutant mice show hyperarousal in the open field test, have impaired social interaction, are less likely to build nests when provided cotton batting and are less likely to bury marbles in the cage bedding. Adult male mice were used for all studies as male FXS patients typically suffer more severe symptoms than do female patients due to the single X chromosome. In both FXS patients and the fmr1 KO mice, there have been found to be alterations in the density, size, shape and maturity of dendritic spines, the principle recipients of excitatory inputs from other neurons (ME Gurney, 2017).

Patients with FXS most frequently have a combination of ADHD and hyperarousal, but other disorders, such as Smith-Magenis syndrome and males with XYY, may have similar volatility of behavior (Hagerman, 1999). Mood problems and anxiety are common in fetal alcohol syndrome (FAS), Williams Syndrome (WS), FXS, Tourette syndrome, and some sex chromosomal disorders, and their identification and psychopharmacological treatment may dramatically enhance the well-being of the patient, and in some cases, significantly reduce aggression or out- bursts (Hagerman, 1999). Lastly, relatively high frequency of significant distortions in thinking on the spectrum of psychotic ideation are being studied in several disorders, including FAS, FXS, velocardiofacial syndrome (VCFS), and Prader- Willi syndrome (PWS), because antipsychotic medication may significantly improve these distortions and overall functioning level.

Multiple studies suggest that variants within the FMR1 gene other than the CGG-repeat expansion mutation can cause dysfunction of FMRP (Suhl, 2015). Similar to the I304N mutation, the G266E mutation is within a conserved amino acid in a KH domain and is very likely to be responsible for the patient's intellectual and behavioral disabilities. The S27X mutation is also very likely to be the root of the patient's symptoms because the truncation is so severe and FMRP is absent in a cell line derived from the patient.

The genetic basis of Autism Spectrum Disorders (ASDs) is highly heterogeneous, as hundreds of different genes have been implicated in their cause. Interestingly, most of the genes show expression profiles at the stage of early development, and their functionalities share strong enrichment in cell adhesion and mobility, cytoskeleton regulation, synapse formation and kinase signaling (Pinto et al., 2010; Gilbert and Man, 2017). These ASD genes include FMR1, LIS1, MECP2, PTEN, SHANK1/2/3, TAOK2, TSC1/2, Neuroligins, Neurexins, KIAA2022/KIDLIA (Gilbert and Man, 2016) and UBE3A/E6- associated protein (E6AP).

FXS patients display a variety of overlapping intellectual deficits with other ASDs ranging from severe cognitive disabilities, autistic behaviors such as aggression, social anxiety and stereotypic acting, attention-deficit hyperactivity disorder, epilepsy and abnormal physical characteristics such as macroorchidism (Hagerman, 1997). FXS and ASD patients show a range of repetitive behaviors, including stereotypies, rituals, compulsions, obsessions and self-injurious. Similar phenotypes occur (but not limited to) in ASD: Angelman Syndrome (AS), Rett Syndrome (RS), Phelan Mcdermid Syndrome (PMS), Pitt Hopkins Syndrome (PTHS).

Efforts to treat FXS have included numerous investigations have not been widely successful, which has led to the exploration for additional and new therapies. Management is symptom-based and requires a multidisciplinary approach. Speech, physical and sensory integration therapy as well as individualized educational plans and behavioral interventions may be combined with medication, such as stimulants for attention deficit-hyperactivity disorder; selective serotonin reuptake inhibitors (SSRIs) for anxiety, depression, obsessive-compulsive disorder; and atypical antipsychotic agents for self-injury and aggressive behaviors. New targeted treatments for FXS are being studied.

An object of the present invention is to overcome one or more of the issues with current treatments for neurological and developmental disorders, such as autism and FXS. A further object of the present invention is to provide treatments for autism mediated by a FMR1 gene mutation. A preferred object of the present invention is to provide treatments for FXS. It would be beneficial if treatments are based on pre-existing pharmaceutically active ingredients.

US 5885976 A discloses a composition useful for treating neurological and mental disorders which are associated with and/or related pathogenetically to deficient serotonin neurotransmission and impaired pineal melatonin functions in humans. The composition increases serotonin transmission to the human to be treated followed by the application to the brain of the human of a sufficient amount of AC pulsed magnetic field alone, or in combination with a DC magnetic field and low frequency random noise, of proper intensity, frequency, waveform, wave symmetry and phase shift of the wave to treat the disorder.

US 9468627B2 discloses the treatment of serotonergic dysregulation disorders and/or augmentation of serotonin levels in the brain by add-on treatments to serotonin enhancers, and slow-release formulations of 5-hydroxytryptophan (5-HTP) therefor.

## Summary of Invention

In accordance with the present invention, there is provided a composition for use in the treatment, management or amelioration of FMR1 mediated autism, wherein the composition comprises one or more tryptophans selected from 5-Hydroxytryptophan (5-HTP) (Oxitriptan), Tryptophan (L-Tryptophan); D-Tryptophan; 2-HydroxyTryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

The FMR1 mediated autism may be due to the FMR1 gene sequence including a mutation comprising one of the following:

a. expansion and subsequent methylation of (CGG)n trinuleotide repeats in the 5'-untranslated region of the FMR1 gene;

b. intragenic point mutations or deletions in the FMR1;

c. a I304N mutation;

d. a G266E mutation; or

e. a S27X mutation.

As used herein, the terms "treatment", "treating", "treat" and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment" as used herein, covers any treatment of a disease in a mammal, particularly in a human, and includes: (a) preventing the disease from occurring in a subject which can be predisposed to the disease but

has not yet been diagnosed as having it; (b) inhibiting the disease, i.e., arresting or slowing its development; and (c) relieving the disease, i.e., causing regression of the disease.

The term "subject" or "individual" used herein includes any human or nonhuman animal. The term "nonhuman animal" includes all mammals, such as nonhuman primates, sheep, dogs, cats, cows, horses.

The one or more tryptophans will preferably comprise 5-Hydroxytryptophan (5-HTP) (Oxitriptan).

Alternatively, the one or more tryptophans may be selected from: Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

The FMR1 mediated autism may be related to Fragile X Syndrome (FXS).

In accordance with a second aspect of the present invention, there is provided a composition for use in the treatment, management or amelioration of Fragile X Syndrome (FXS), wherein the composition comprises one or more tryptophans selected from 5-Hydroxytryptophan (5-HTP) (Oxitriptan), Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

The one or more tryptophans will preferably comprise 5-Hydroxytryptophan (5-HTP) (Oxitriptan).

Alternatively, the one or more tryptophans may be selected from: Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

The skilled addressee will understand that the optimum dose of the composition will need to be established for both the first and second aspects. However, it is preferred that the composition is administered in a daily dose in the range of about 20 mg and about 400 mg. In some embodiments, the composition is administered in a daily dose in the range of about 100 mg and about 800 mg, suitably in a daily dose in the range of about 200 mg to about 400 mg, for example a daily dose of approximately 300 mg or a daily dose of 300 mg.

The daily dose of the composition may be administered in a single daily dose. Suitably the daily dose is administered in one to five daily doses, suitably in two to four daily doses or in three daily doses. In some embodiments, the composition comprising one or more tryptophans is administered in a dose of 100 mg TID (ter in die / three times a day) and therefore a total dose of 300 mg per day, for example at approximately 8 hour intervals.

The composition comprising one or more tryptophans may consist essentially or consist of tryptophans, as defined above. Suitably the composition consists essentially or consists of a tryptophan, for example oxitriptan. The present invention may therefore provide tryptophans, for example oxitriptan, for use in the treatment, management or amelioration of FMR1 mediated autism, suitably wherein the treatment involves administering to a patient in need thereof a daily dose of the tryptophans, for example oxitriptan, of from 100 mg and about 800 mg, suitably in a daily dose in the range of about 200 mg to about 400 mg, for example a daily dose of approximately 300 mg or a daily dose of 300 mg. Preferably in a dose of 100 mg TID.

In one embodiment, the composition for use of the first or second aspects of the present invention, may further comprise one or more ergot alkaloids.

The ergot alkaloid may comprise ergoloid mesylates. The ergot alkaloid may be selected from one or more of the following: methysergide; dihydroergotamine; lisuride ergotamine nicergoline; dihydroergocristine; dihydroergocornine; dihydroergocryptine; ergometrine; methylergometrine; cabergoline; pergolide; bromocriptine; lysergic acid diethylamide; terguride; and metergoline. Preferably, the ergot alkaloid comprise a substantially equiproportional preparation of dihydroergocornine, dihydroergocristine, and dihydroergocryptine.

The one or more tryptophans are suitably administered in the doses described above. In embodiments wherein the composition comprises one or more ergot alkaloids, the one or more ergot alkaloids are suitably administered in a daily dose in the range of about 1 to 10 mg, suitably from 1 to 5 mg, suitably from 2 to 4 mg, for example around 3 mg per day or 3 mg per day. In some embodiments, the composition is administered in a daily dose in the range of about 3 mg and about 5 mg.

The daily dose of the one or more ergot alkaloids described above may be administered in a single daily dose. Suitably the daily dose is administered in one to five daily doses, suitably in two to four daily doses or in three daily doses. In some embodiments, the composition comprising one or more ergot alkaloids is administered in a dose of 1 mg TID (ter in die / three times a day) and therefore a total dose of 3 mg per day, for example at approximately 8 hour intervals.

Suitably these daily doses are of ergoloid mesylates.

The one or more ergot alkaloids may comprise ergoloid mesylates. The one or more ergot alkaloids may consist essentially or consist of ergoloid mesylates.

In some embodiments, the one or more tryptophans are administered in a dose of 100 mg TID and the one or more ergot alkaloids are administered in a dose of 1 mg TID, suitably wherein the one or more tryptophans is oxitriptan and the one or more ergot alkaloids is ergoloid mesylates.

Suitably the composition comprises oxitriptan and ergoloid mesylates and is administered in a dose of about 100 mg TID of oxitriptan and a dose of about 1 mg TID of ergoloid mesylates.

In formulation, the one or more tryptophans and the one or more ergot alkaloids may be in a mixture. Such a mixture may be a formulation where both components are interspersed with one another. Alternatively, each component can be separated in the same dose.

The one or more tryptophans may be for administration separately, together or sequentially with the one or more ergot alkaloids.

The composition may comprise oxitriptan and an ergoloid mixture, wherein the composition is administered in a daily dose in the range of about 20 mg to about 400 mg of oxitriptan and in the range of about 1 mg to about 3mg of ergoloid mixture.

In all embodiments, the ergot alkaloid may comprise ergoloid mesylates.

Ergoloid mesylates [https://www.drugbank.ca/drugs/DB01049] is an equiproportional preparation of three different ergotamantriones: dihydroergocornine, dihydroergocristine, and dihydroergocryptine [Thompson 1990]. All these components are produced by the fungus Claviceps purpurea and are all derivatives of the tetracyclic compound 6-methylergonovine [Pillay 2013] The derivatives of this fungus are identified to be about 350 different substances from which the components of the ergoloid mesylates mixture are composed of the dihydrogenated ergot alkaloid derivatives [PERCHESON 1954]. The mixture of ergoloid mesylates was first developed by Novartis and The United States Food and Drug Administration (FDA) approved on November 5, 1953, but this specific formulation is now discontinued [https://www.accessdata.fda.gov/scripts/cder/daf/index.cfm?event=overview.process &ApplNo=009087]. Later in 1991, the mixture of ergoloid mesylates was retaken by Sun Pharmaceutical Industries and approved by the FDA [https://www.accessdata.fda.gov/scripts/cder/daf/index.cfm?event=overview.process &ApplNo=009087].

Ergoloid mesylates has known mechanism involving dopamine, serotonin, alpha and beta adrenergic receptor protein groups. It has a predicted bioactivity with OPRM1. Ergoloid mesylates has a reported plasma half-life of 3.5 hours while the terminal half-life is of 13 hours [Seyffart 1992]. Ergoloid mesylates has a suggested trial adult dosage of 1.5 (1-3) mg per day in 3x0.5-0.6mg every 8 hrs.

Preferably, the ergot alkaloid is selected from one or more of the components of the ergoloid mesylates mixture: epicriptine, dihydro-alpha-ergocryptine, dihydroergocornine, and dihydroergocristine. In certain embodiments, the ergot alkaloid comprises one of the components of the ergoloid mesylates mixture selected from: epicriptine, dihydro-alpha-ergocryptine, dihydroergocornine, and dihydroergocristine. In other embodiments, the ergot alkaloid comprises two or more selected from: epicriptine, dihydro-alpha-ergocryptine, dihydroergocornine, and dihydroergocristine. In alternative embodiments, the ergot alkaloid comprises a mixture of epicriptine, dihydro-alpha-ergocryptine, dihydroergocornine, and dihydroergocristine.

A range of tryptophans may be used in conjuction with the present invention.

The tryptophan may comprise 5-Hydroxytryptophan (5-HTP) (Oxitriptan).

5-Hydroxytryptophan (5-HTP), also known as oxitriptan (INN) [https://www.drugbank.ca/drugs/DB02959], is a naturally occurring amino acid and metabolic intermediate in the synthesis of serotonin and melatonin. 5-HTP is sold overthe-counter in the United Kingdom, United States and Canada as a dietary supplement for use as an antidepressant, appetite suppressant, and sleep aid, and is also marketed in many European countries for the indication of major depression under trade names like Cincofarm, Levothym, Levotonine, Oxyfan, Telesol, Tript-OH, and Triptum. Several double-blind placebo-controlled clinical trials have demonstrated the effectiveness of 5-HTP in the treatment of depression, though a lack of high quality studies has been noted. More study is needed to determine efficacy in treating depression.

5-Hydroxytryptophan is suitably in its active L form or comprises the active L form, and may also be known as 5-hydroxy-L-trytophan or 5-hydroxytryptophan L-form. The IUPAC name for 5-Hydroxytryptophan (5-HTP) (oxitriptan) is 2-amino-3-(5-hydroxy-1H-indol-3-yl)propanoic acid.

In some embodiments the 5-Hydroxytryptophan is in the D form and may also be known as 5-hydroxy-D-trytophan or 5-hydroxytryptophan D-form.

The body produces 5-Hydroxytryptophan (5-HTP) from the essential amino acid L-tryptophan (LT). 5-HTP is extracted from the seeds of the African plant, Griffonia simplicifolia. Typically, 5-HTP is the rate limiting step of conversion of LT to serotonin. Serotonin levels regulate sleep, mood, appetite, temperature, pain, and aggressive or sexual behaviors. 5- HTP is found in many commercial combination products and also used alone for sleep onset, depression, anxiety, and to decrease appetite.

The known mechanism of action for 5-Hydroxytryptophan is trpS2. It has predicted bioactivity against ESR1, HTR1A, GRIA1, GRIK1. 5-Hydroxytryptophan has a half life of 2 hrs (Jacobsen JPR 2016) and a suggested trial adult dosage of 60 (20-400) mg per day in 3x20 mg every 8 hrs.

The skilled addressee will readily understand that additional compounds in the tryptophan category is expected to invoke similar phenotypic effects and could be employed in conjunction with the present invention.

In certain embodiments, the one or more tryptophans may be selected from: Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide. The one or more tryptophans may be a single tryptophan or a

mixture of two or more of the tryptophans.

Also disclosed but not forming a part of the present invention is a composition according to any aspect of the invention for use in the treatment, management or amelioration of a range of autism diseases or diseases other than FMR1 mediated autism or Fragile X Syndrome (FXS) where autism is a known component.

The disease may be selected from one or more of the following rare diseases with associated autism: 1p21.3 microdeletion syndrome; adenylosuccinate lyase deficiency; autism-facial port-wine stain syndrome; autism spectrum disorder due to AUTS2 deficiency; autism spectrum disorder-epilepsy-arthrogryposis syndrome; developmental delay with autism spectrum disorder and gait instability; inverted duplicated chromosome 15 syndrome; macrocephaly-intellectual disability-autism syndrome; severe neurodevelopmental disorder with feeding difficulties-stereotypic hand movement-bilateral cataract; Smith-Magenis syndrome; tuberous sclerosis complex; Xq12-q13.3 duplication syndrome;

1p21.3 microdeletion syndrome ORPHA:293948 is an extremely rare chromosomal anomaly characterized by severe speech and language delay, intellectual deficiency, autism spectrum disorder. Clinical description: 1p21.3 microdeletion syndrome is characterized by severe speech and language delay, a borderline-mild to mildmoderate intellectual deficiency, autism spectrum disorder features, and minor dysmorphic facial features such as long ears, deep set eyes, a broad nasal tip and a thick lower lip. Affected individuals have normal gross motor development without major abnormalities, they are often very shy and friendly with a tendency to overeat.

Adenylosuccinate lyase deficiency ORPHA:46 is a disorder of purine metabolism characterized by intellectual disability, psychomotor delay and/or regression, seizures, and autistic features. Clinical description: ADSL covers a continuous clinical spectrum with three major forms: fatal neonatal, severe (type I), and mild to moderate form (type II). Clinical variability is found, even in patients from the same family. Onset is generally between birth and early childhood. Cases ranging from fatal neonatal encephalopathy (presenting with hypokinesia, intractable seizures and respiratory failure) to mild intellectual disability have been reported. Intellectual disability is found in all patients, epilepsy of various types in most, and autistic features in about one third (failure to make eye contact, hypersensitivity to noise and light, repetitive behavior, agitation, temper tantrums, autoaggression and self-mutilation). Other less common manifestations include psychomotor delay, hyperactivity, speech impairment, muscular hypotonia, muscle wasting, and spasticity. Severely affected patients often have microcephaly. Prenatal manifestations are also reported: impaired intrauterine growth, microcephaly, fetal hypokinesia, and loss of fetal heart rate variability.

Autism-facial port-wine stain syndrome ORPHA:137911 is characterised by the presence of a unilateral angioma on the face and autistic developmental problems characterised by language delay and atypical social interactions.

Autism spectrum disorder due to AUTS2 deficiency ORPHA:352490 is a rare genetic syndromic intellectual disability characterized by global developmental delay and borderline to severe intellectual disability, autism spectrum disorder with obsessive behavior, stereotypies, hyperactivity but frequently friendly and affable personality, feeding difficulties, short stature, muscular hypotonia, microcephaly, characteristic dysmorphic features (hypertelorism, high arched eyebrows, ptosis, deep and/or broad nasal bridge, broad/prominent nasal tip, short and/or upturned philtrum, narrow mouth, and micrognathia), and skeletal anomalies (kyphosis and/or scoliosis, arthrogryposis, slender habitus and extremities). Other clinical features may include hernias, congenital heart defects, cryptorchidism and seizures.

Autism spectrum disorder-epilepsy-arthrogryposis syndrome ORPHA:370943 is a form of congenital disorders of N-linked glycosylation characterized by distal arthrogryposis (mild flexion contractures of the fingers, deviation of the distal phalanges, swan-neck deformity), retromicrognathia, general muscle hypotonia, delayed psychomotor development, autism spectrum disorder (speech delay, abnormal use of speech, difficulties in initiating, understanding and maintaining social interaction, limited nonverbal communication and repetitive behavior), seizures, microcephaly and mild to moderate intellectual disability that becomes apparent with age. The disease is caused by mutations in the gene SLC35A3 (1p21).

Developmental delay with autism spectrum disorder and gait instability ORPHA:329195 is a rare, genetic, neurological disorder characterized by infant hypotonia and feeding difficulties, global development delay, mild to moderated intellectual disability, delayed independent ambulation, broad-based gait with arms upheld and flexed at the elbow with brisk walking or running, and limited language skills. Behavior patterns are highly variable and range from sociable and affectionate to autistic behavior.

Inverted duplicated chromosome 15 syndrome ORPHA:3306 is a rare, complex chromosomal duplication/inversion in the region 15q11.2-q13.1 characterized by early central hypotonia, global developmental delay and intellectual deficit, autistic behavior, and seizures. Clinical description: Presentation is typically with neonatal hypotonia, feeding difficulties and gross motor delay. Global developmental delay is typical in early childhood with speech and language particularly affected. Expressive language is absent or very poor and often echolalic. Comprehension is very limited and contextual. Intention to communicate is absent or very limited. Most children and adults have moderate to severe intellectual disability. The distinct behavioral disorder manifesting in children and adolescents has been widely described as autistic or autistic-like. Seizures occur in over half of affected individuals, with onset typically between 6 months and 9 years, and may include infantile spasms and myoclonic, tonicclonic, tonic, atonic, atypical absences, and focal seizures. Various EEG (electroencephalography) abnormalities have been described. Muscle hypotonia is observed in almost all

individuals, associated, in most cases, with joint hyperextensibility and drooling. Facial dysmorphism is absent or subtle, and major malformations are rare.

Macrocephaly-intellectual disability-autism syndrome ORPHA:210548 is a rare, genetic, neurological disease characterized by association of macrocephaly, dysmorphic facial features and psychomotor delay leading to intellectual disability and autism spectrum disorder. Facial dysmorphism may include frontal bossing, hypertelorism, midface hypoplasia, depressed nasal bridge, short nose, and long philtrum.

Severe neurodevelopmental disorder with feeding difficulties-stereotypic hand movement-bilateral cataract ORPHA:500545 is a rare pervasive developmental disorder characterized by microcephaly, profound developmental delay, intellectual disability, bilateral cataracts, severe epilepsy including infantile spasms, hypotonia, irritability, feeding difficulties leading to failure to thrive, and stereotypic hand movements. The disease manifests in infancy. Brain imaging reveals delay in myelination and cerebral atrophy.

Smith-Magenis syndrome ORPHA:819 is a complex genetic disorder characterized by variable intellectual deficit, sleep disturbance, craniofacial and skeletal anomalies, psychiatric disorders, and speech and motor delay. Clinical description: Patients have a recognizable clinical picture. Craniofacial features include brachycephaly, frontal bossing, hypertelorism, synophrys, upslanting palpebral fissures, midface hypoplasia, a broad square-shaped face with depressed nasal bridge, an everted upper lip with a "tented" appearance, and micrognathia in infancy. Dental anomalies include tooth agenesis and taurodontism. Short stature is common in young patients, with height typically in the normal range as adults. Excess weight and/or obesity in teens and adults are common. Other skeletal anomalies include brachydactyly, scoliosis, 5thfinger clinodactyly, 2/3 toe syndactyly, forearm and elbow limitations, vertebral anomalies, persistent fetal finger pads, and polydactyly. Otolaryngological problems such as velopharyngeal insufficiency, a hoarse deep voice, and vocal cord nodules and polyps are also common; hearing loss (60% of patients) is variable and may be mild to moderate. Ophthalmologic features (>60%) include myopia and iris anomalies and rarely, retinal detachment (often resulting from violent behaviors). Mild to moderate intellectual deficit, significant speech delay, decreased sensitivity to pain, peripheral neuropathy, as well as characteristic sleep disturbances and maladaptive behaviors (outbursts/temper tantrums, attention seeking, aggression, disobedience, distraction, and self-injurious behaviors) are common. Organ malformations (30-40%) include cardiac, renal, urinary tract, and central nervous system (CNS) abnormalities.

Tuberous sclerosis complex (TSC) ORPHA:805 is a neurocutaneous disorder characterized by multisystem hamartomas and associated with neuropsychiatric features. Clinical description: TSC is characterized by multisystem hamartomas, most commonly skin, brain, kidney, lung and heart, appearing at different ages. Skin involvement includes: hypomelanotic macules (ash leaf) present within the first years of life; angiofibromas that appear at age 3-4 years as erythematous and papulonodular lesions; ungual fibromas; cephalic and lumbar (shagreen patch) fibrous plaques; and "confetti" skin lesions appearing in childhood to early adolescence. Brain is involved in almost all cases of TSC, with the presence of different neuropathological lesions, such as cortico/subcortical tubers, radial migration lines, subependymal nodules, SEGA. SEGA can cause hydrocephalus (growth risk higher in the first 3 decades). Early-onset epilepsy (infantile spasms and/or focal seizures) is present in 85% of patients. Neuropsychiatric features (intellectual disability, attention-deficit/hyperactivity disorder, autism spectrum disorders (ASD), self-injury, anxiety and obsessive compulsive tendencies have also been reported. Renal angiomyolipomas (AML) develop during childhood with a higher risk of growth during adolescence and adulthood and manifest by pain, hematuria/retroperitoneal hemorrhage, abdominal masses, hypertension and renal failure. Lymphangioleiomyomatosis (LAM), multifocal micronodular pneumocyte hyperplasia (MMPH) and pulmonary cysts develop during adulthood and manifest with dyspnea, pneumothorax, or chylothorax. Cardiac rhabdomyomas (CR) appear during the fetal period and may become symptomatic (outflow tract obstruction or by interfering with valvular function) during infancy and early childhood. Additional features include dental enamel pitting, intraoral fibromas and skeletal dysplasias.

Xq12-q13.3 duplication syndrome ORPHA:314389 is a rare chromosomal anomaly syndrome, resulting from the partial duplication of the long arm of chromosome X, characterized by global developmental delay, autistic behavior, microcephaly and facial dysmorphism (including down-slanting palpebral fissures, depressed nasal bridge, anteverted nares, long philtrum, down-slanting corners of the mouth). Seizures have also been reported in some patients.

The disease may be selected from one or more of the following autism spectrum disorders (pervasive developmental disorders): Asperger syndrome, atypical autism and autistic disorder.

Asperger syndrome is an autism spectrum disorder that is characterized by significant difficulties in social interaction, along with restricted and repetitive patterns of behavior and interests. It differs from other autism spectrum disorders by its relative preservation of linguistic and cognitive development.

Atypical autism is an autism spectrum disorder that involves some autistic symptoms occuring after age 3 with an abscence of all the traits necessary for a diagnosis of autism.

Autistic disorder is an autism spectrum disorder that is characterized by symptoms across all three symptom domains (communication, social, restricted repetitive interests and behaviors), delayed language development, and symptom onset prior to age 3 years.

The autism spectrum disorder may have overlapping phenotypes, such as Angelman Syndrome (AS), Rett

Syndrome (RS), Phelan Mcdermid Syndrome (PMS), Pitt Hopkins Syndrome (PTHS).

The disease may be selected from one or more of the following behavioral disorders: attention deficit and hyperactivity disorder; stereotypic movement disorder; conduct disorder; generalized anxiety disorder; neurotic disorder; obsessive-compulsive disorder; agoraphobia; social phobia; and separation anxiety disorder and 15q11q13 microduplication syndrome.

Attention Deficit and Hyperactivity Disorder is a specific developmental disorder that is characterized by coexistence of attentional problems and hyperactivity, with each behavior occurring infrequently alone and symptoms starting before seven years of age.

Stereotypic movement disorder is a specific developmental disorder that is characterized by repeated, rhythmic, purposeless movements or activities such as head banging, nail biting, or body rocking.

Conduct disorder is a specific developmental disorder marked by a pattern of repetitive behavior wherein the rights of others or social norms are violated.

Generalized anxiety disorder is an anxiety disorder that is characterized by long-lasting anxiety that is not focused on any one object or situation.

Neurotic disorder is an anxiety disorder that involves distress but neither delusions nor hallucinations.

Obsessive-compulsive disorder is an anxiety disorder that involves unwanted and repeated thoughts, feelings, ideas, sensations (obsessions), or behaviors that make them feel driven to do something (compulsions).

Agoraphobia is a phobic disorder involving the specific anxiety about being in a place or situation where escape is difficult or embarrassing or where help may be unavailable.

Social phobia is a phobic disorder that involves social anxiety occurring only in specific public or social situations, interactions with others or being evaluated or scrutinized by other people.

Separation anxiety disorder is an anxiety disorder that involves the feeling of excessive and inappropriate levels of anxiety over being separated from a person to whom the individual has a strong emotional attachment or place.

Other behavioural disorders may be impeded social interaction (such as poor eye contact or solitude preference), communication or language problems (such as speech delay or pretense of deafness), repetitive and/or obsessive behavior (such as stereotyped behavior or extreme restlessness), signs of memory loss and signs of disruptive behaviour.

Features, integers, characteristics, compounds, molecules, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and figures), and/or all of the steps of any method or process so disclosed, may be combined in any combination, except combinations where at least some of such features and/or steps are mutually exclusive. The invention is set out in the appended set of claims.

In some embodiments, the composition for use in the treatment, management or amelioration of FMR1 mediated autism of this first aspect or the composition for use in the treatment, management or amelioration of Fragile X Syndrome (FXS), may involve administering the composition to an individual who is already receiving a treatment of other compounds and/or compositions. Suitably the individual is already receiving a selective serotonin re-uptake inhibitor (SSRI), for example fluvoxamine. Individual who may benefit from treatment with the compositions of the present invention may be likely to already be receiving an SSRI compound as a treatment for autism or other disorder. Therefore the compositions of the present invention may advantageously be coadministered with and be efficacious in the presence of an SSRI in the treatment, management or amelioration of an autism disease or disease where autism is a known component, for example FMR1 mediated autism.

Therefore the present invention may provide a combination of an SSRI and a composition comprising one or more tryptophans and optionally one or more ergot alkaloids for use in the treatment, management or amelioration of an autism disease or disease where autism is a known component, for example FMR1 mediated autism. In such embodiments, the SSRI may be administered in the typical daily dose for that SSRI and the composition comprising one or more tryptophans and optionally one or more ergot alkaloids may be administered in the daily doses discussed above. Suitably in such embodiments the composition comprising one or more ergot alkaloids is ergoloid mesylates.

## Detailed Description of the Invention

Embodiments of the invention are described below, by way of example only with reference to and as illustrated in the following figures:

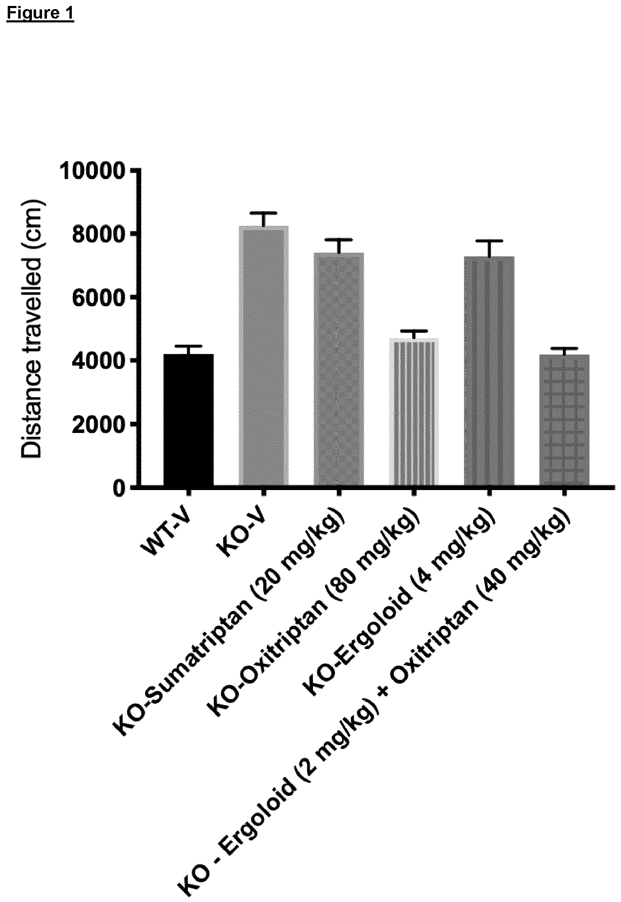

Figure 1 is a bar graph showing the open field WT-V, KO-V, Sumatriptan, Oxitriptan, Ergoloid and combinations (Ergoloid and Sumatriptan, Ergoloid and Oxitriptan);

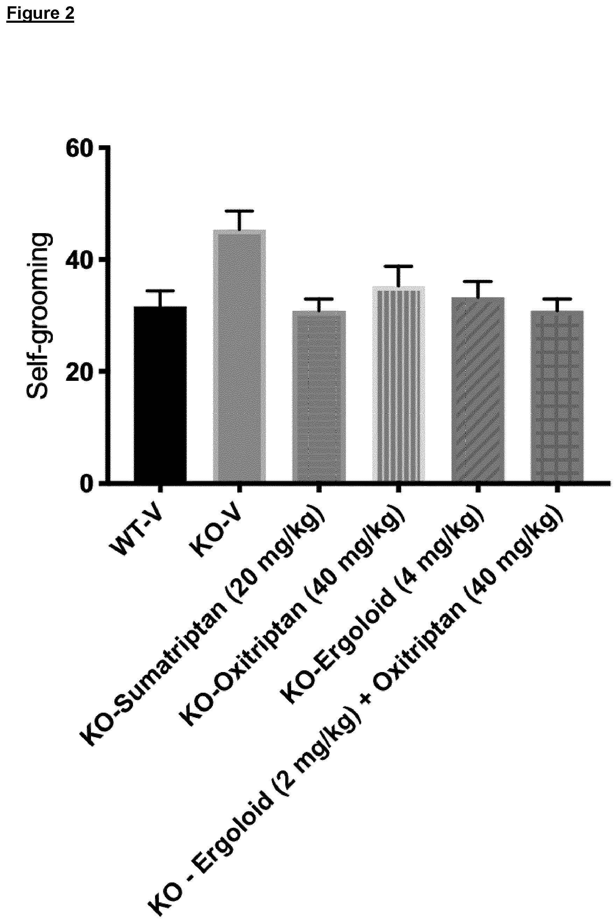

Figure 2 is a bar graph showing the stereotypy WT-V, KO-V, Sumatriptan, Oxitriptan, Ergoloid and combinations (Ergoloid and Sumatriptan, Ergoloid and Oxitriptan);

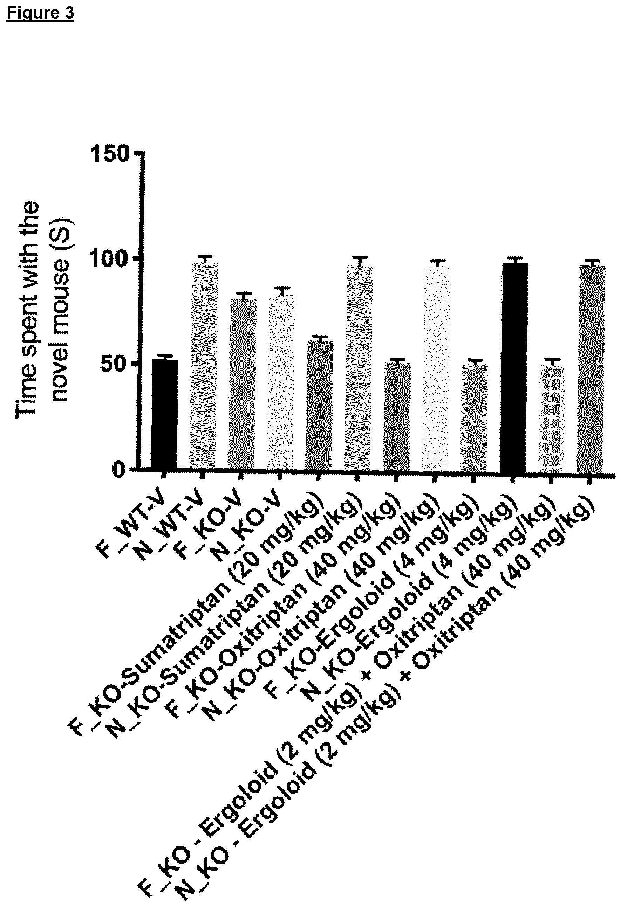

Figure 3 is a bar graph showing sociability WT-V, KO-V, Sumatriptan, Oxitriptan, Ergoloid and combinations (Ergoloid and Sumatriptan, Ergoloid and Oxitriptan);

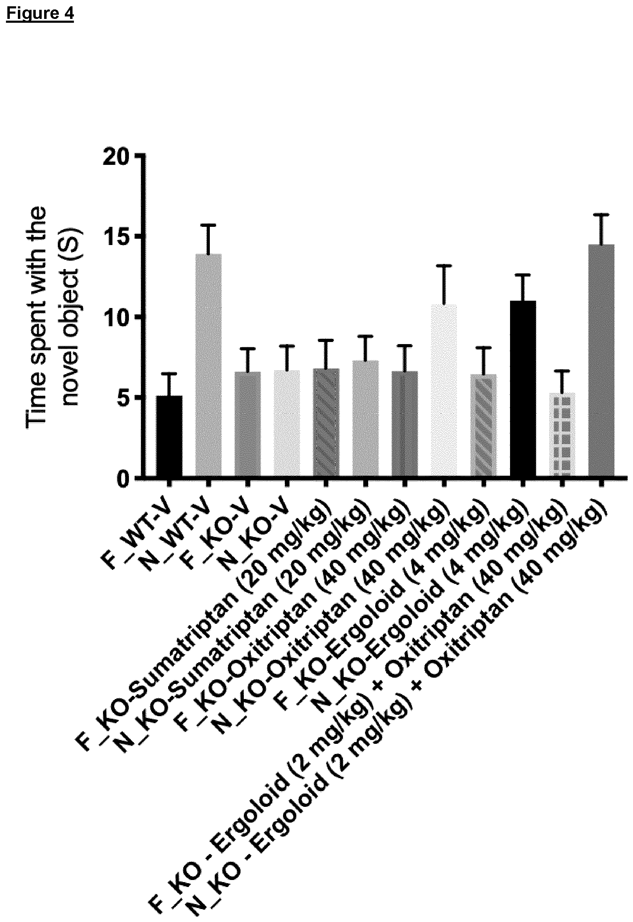

Figure 4 is a bar graph showing Novel Object Recognition (NOR) WT-V, KO-V, Sumatriptan, Oxitriptan, Ergoloid and combinations (Ergoloid and Sumatriptan, Ergoloid and Oxitriptan);

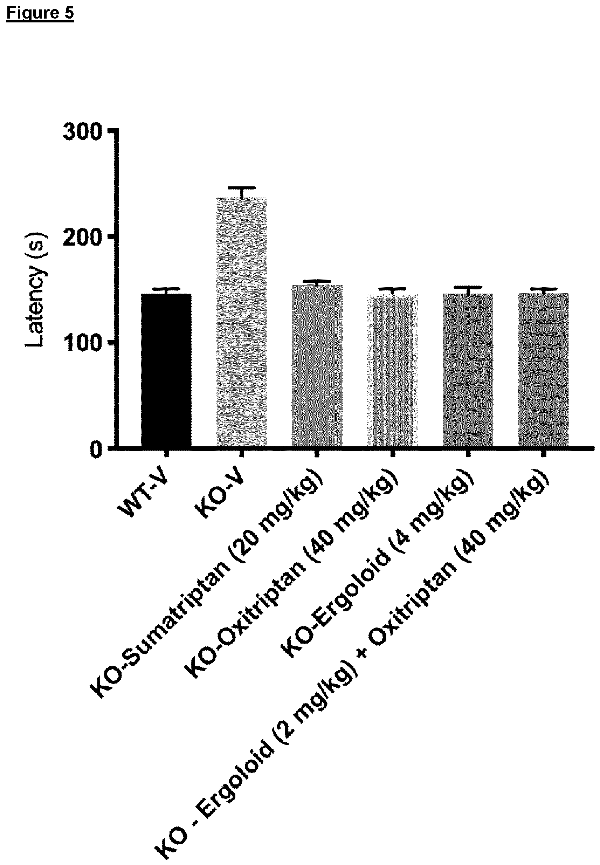

Figure 5 is a bar graph showing hyponeophagia WT-V, KO-V, Sumatriptan, Oxitriptan, Ergoloid and combinations (Ergoloid and Sumatriptan, Ergoloid and Oxitriptan); and

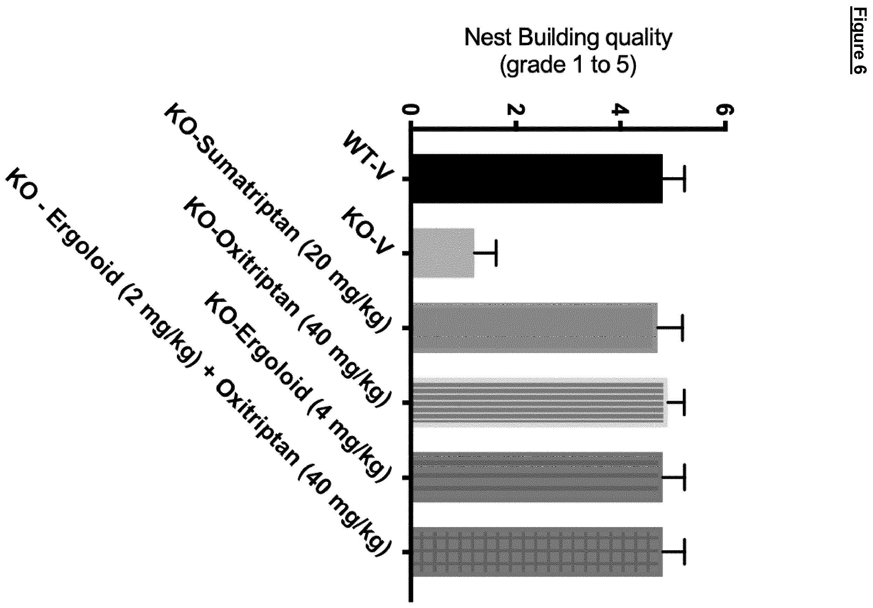

Figure 6 is a bar graph showing test of daily living WT-V, KO-V, Sumatriptan, Oxitriptan, Ergoloid and combinations (Ergoloid and Sumatriptan, Ergoloid and Oxitriptan).

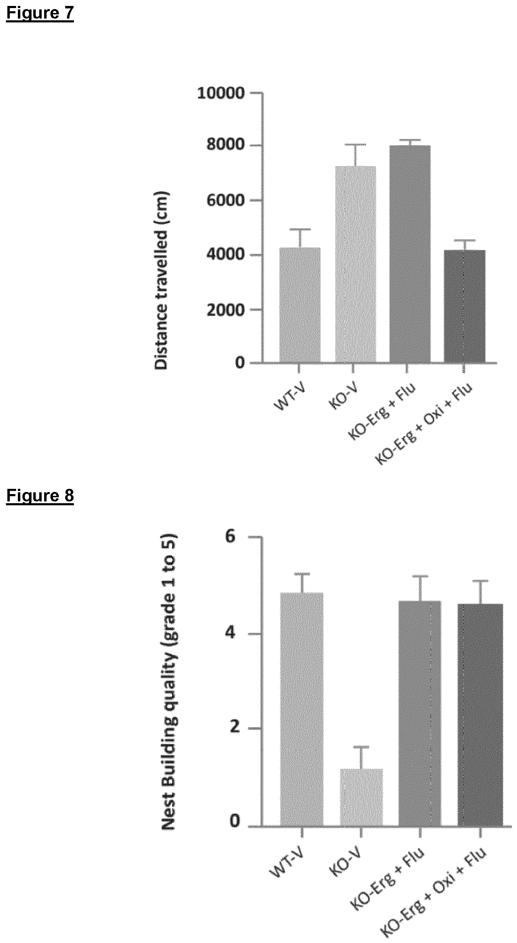

Figure 7 is a bar graph showing the open field outcomes for WT-V, KO-V, Ergoloid/Fluvoxamine combination, and Ergoloid/Oxitriptan/Fluvoxamine combination.

Figure 8 is a bar graph showing the nesting outcomes for WT-V, KO-V, Ergoloid/Fluvoxamine combination, and Ergoloid/Oxitriptan/Fluvoxamine combination.

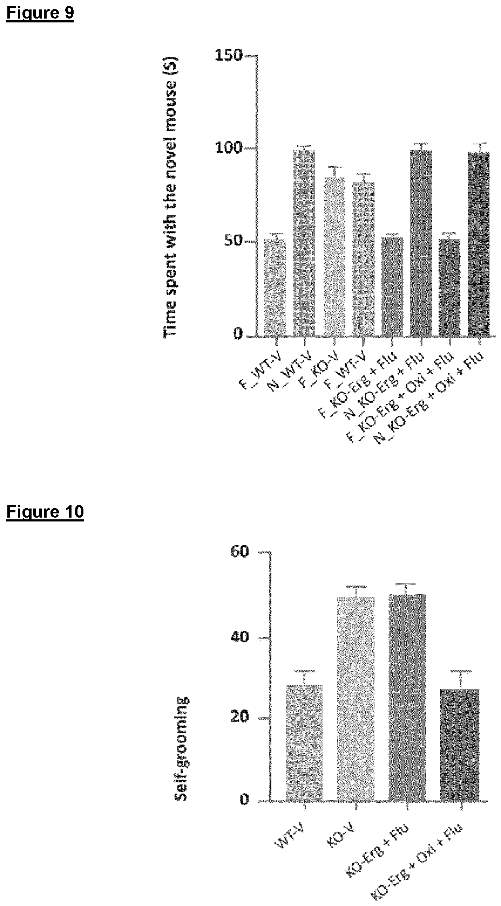

Figure 9 is a bar graph showing the sociability outcomes for WT-V, KO-V, Ergoloid/Fluvoxamine combination, and Ergoloid/Oxitriptan/Fluvoxamine combination.

Figure 10 is a bar graph showing the stereotypy outcomes for WT-V, KO-V, Ergoloid/Fluvoxamine combination, and Ergoloid/Oxitriptan/Fluvoxamine combination.

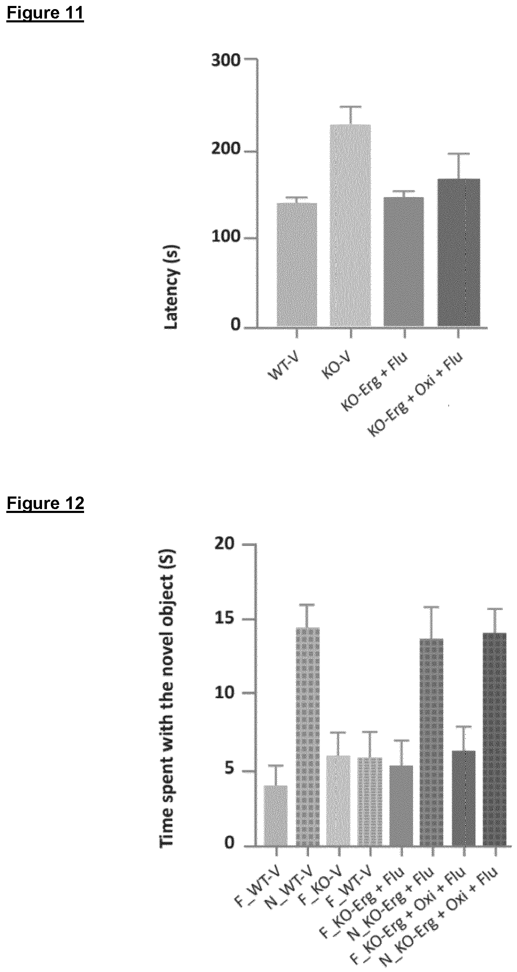

Figure 11 is a bar graph showing the hyponeophagia outcomes for WT-V, KO-V, Ergoloid/Fluvoxamine combination, and Ergoloid/Oxitriptan/Fluvoxamine combination.

Figure 12 is a bar graph showing the NOR outcomes for WT-V, KO-V, Ergoloid/Fluvoxamine combination, and Ergoloid/Oxitriptan/Fluvoxamine combination.

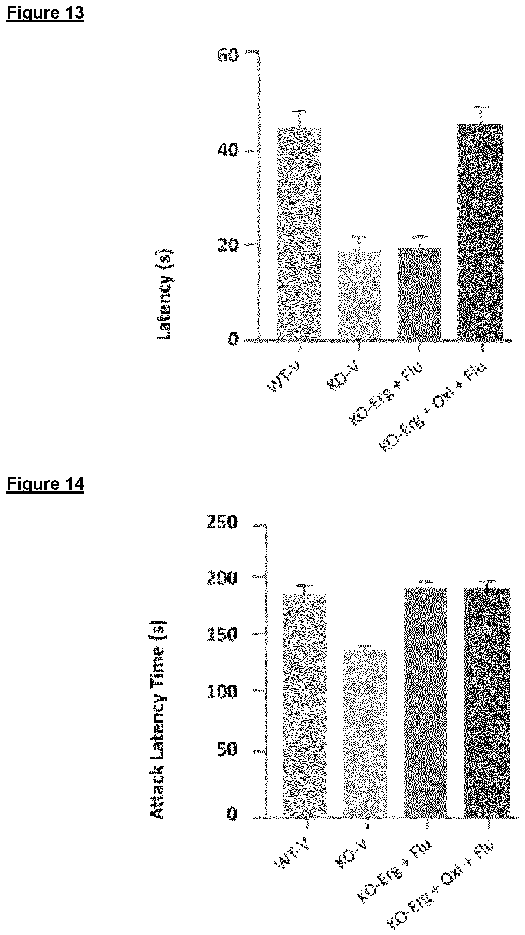

Figure 13 is a bar graph showing the fear conditioning outcomes for WT-V, KO-V, Ergoloid/Fluvoxamine combination, and Ergoloid/Oxitriptan/Fluvoxamine combination.

Figure 14 is a bar graph showing the resident intruder outcomes for WT-V, KO-V, Ergoloid/Fluvoxamine combination, and Ergoloid/Oxitriptan/Fluvoxamine combination.

## Examples

### **Example 1** - **Studies relating ergoloid mesylates, oxitriptan and sumatriptan to phenotypic effects in FXS and ASDs**

Tryptophan has been shown to reduce the intensity and duration of migraine headaches (Titus et al., 1986). However, some controversial results were reported from a group of patients that were administered an amino acid drink which contained L-tryptophan (Drummond, 2006). The later study suggests that a reduction in brain synthesis of serotonin intensifies photophobia and other migrainous symptoms and thus might contribute to the pathogenesis of migraine.

Hawkins (2020) reported a case of a 15-year-old male with autism and a lifelong history of severe insomnia which was treated with 5-HTP since the age of 5 years. Typical doses of 5- HTP for insomnia are 50-200mg given in the evening. 5-HTP has been shown to stabilize sleep schedule and increase REM sleep.

Additionally, it has been shown that the levels of the amino acid tryptophan, the precursor of serotonin, is lower than normal in autistic brains, and that a diet poor in tryptophan worsens autistic symptoms (Boccuto et al., 2013).

It was shown that the stimulation of 5-HT7 serotonin receptors in post- synaptic compartments reverses mGluR-LTD in hippocampal slices of FXS mouse brains, suggesting that 5-HT7 receptor agonists might be envisaged as novel therapeutic tools for FXS (Costa et al., 2012).

These same authors characterized two new molecules with very high binding affinity and selectivity for 5-HT7 receptors and ability to rescue exaggerated mGluR-LTD that might be used as novel pharmacological tools for the therapy of FXS (Costa et al., 2015).

Increasing serotonergic signaling can potentially rescue the neurobiology that is disrupted in FXS by upregulating levels of BDNF, increasing the number of GluA1 receptors and GlutA1-LTP, increasing levels of serotonin in the

synapse, and by enhancing the dopaminergic system. These mechanisms are thought to improve synaptic plasticity and brain development. Other effects may include balancing cortical asymmetry of serotonin and overall neuroprotective effects (Hanson & Hagerman, 2014).

The possibility of accelerated serotonin metabolism in the autistic syndrome has been studied by Ritvo et al. (1971). These investigators administered L-dopa to four autistic children in an attempt to produce clinical improvement by lowering blood concentrations of 5-HT. Although concentrations of 5-HT were significantly decreased, no change in behavior was observed. The findings of the present study in conjunction with those of Ritvo et al. (1971) do not offer encouragement that autistic children are likely to benefit from therapies based upon the manipulation of 5-HT metabolism (Sverd et al., 1978).

Autistic patients have a greater response to sumatriptan than do normal controls independent of placebo effects. Also, in patients with autism or Asperger's disorder, GH response to sumatriptan is significantly greater than to placebo, in contrast to a more moderate difference in sumatriptan vs. placebo GH response in normal controls. This suggests that in autistic patients, 5-HT dysfunction may reflect hypersensitivity of the inhibitory 5-HT1d receptor. These findings are consistent with previous findings of decreased 5-HT synthesis in the frontal and thalamic brain regions of patients with autism or Asperger's disorder (Novotny et al., 2000).

Results show that the severity of repetitive behaviors (as measured by the YBOCS-compulsion subscale), but not other behavioral dimensions (communication and social deficits as measured by ADI-R algorithm subscales), parallels sumatriptan-elicited growth hormone response. This suggests that a specific component of the 5HT system (the 5HT 1d receptor) may play a role in mediating one specific behavioral component of autistic disorder (repetitive behavior), thus influencing heterogeneity in autism (Hollander et al., 2000).

*Animal testing*

Fmr1 knockout mice recapitulate the human phenotype and represent a valuable preclinical model for assessment of putative drug treatments. More than 20 years ago, a first animal model was described, the Fmr1 knockout (KO) mouse. The Fmr1 KO carries an insertion in exon 5 (Bakker et al., 1994). It is a protein null, although Fmr1 mRNA is still present (Yan et al., 2004). These mice have been backcrossed to the C57/BI6 or the FVB strains. The Fmr1 KO2 is a null allele at Fmr1 generated by deletion of the promoter and first exon of Fmr1 (Mientjes et al., 2006). It is both protein and mRNA null. This mutation is the same as is produced by Cre-mediated excision of the loxP sites present in the Fmr1 cKO described below (we house these and other mice models of FXS).

Impaired inhibitory regulation of GSK3 in Fmr1 knockout mice may contribute to some socialization deficits and that lithium treatment can ameliorate certain socialization impairments (Mines et al., 2010). The Fmr1 KO mouse might be useful to study some social aspects of ASD, particularly when hyperactivity coexists (Sørensen et al., 2015).

Fragile X Syndrome has a symptomatology resembling autism to a very large extent and the validated genetic mouse model that is available for this disorder, the Fmr1 KO mouse, also shows much promise as a possible model for autism (Bernadet & Crusio, 2006).

MeCP2 mRNA was identified as a substrate for FMRP. This X-linked MeCP2 gene is mutated in RS, another neurodevelopmental disorder associated with autistic features. Levels of MeCP2 protein were elevated in null-treated Fmr1 KO mouse brains (Arsenault et al., 2016).

mGluR5 stimulated protein synthesis of alphaCaMKII and PSD-95 are impaired in synaptoneurosomes from Fmr1 KO mice. Furthermore, CAMKII dependent phosphorylation of MeCP2 links these synaptic proteins to RS, another single gene disorder associated with autism, and transcriptional regulation of brain derived nerve growth factor (BDNF). Results suggest autism to be a synapsopathy disease where disruption of the synapse during development produces a common clinical picture, despite a heterogeneity of interconnected causes. The later suggests that treatments for fragile X, may have efficacy in treating other causes of autism (Dölen & Bear, 2009).

Adult Fmr1 KO mice showed decreased baseline gene expression of select cytokines in the hippocampus compared with WT mice. Proinflammatory cytokines IL-6 and TNF-$\alpha$ were significantly decreased in Fmr1 KO mice. Proinflammatory cytokines are involved in the amplification of many inflammatory reactions and downstream CNS signaling cascades that have the ability to affect cognition and behavior (Hodges et al., 2017).

The layer 4 network in the Fmr1-KO exhibits significant alterations in spike output in response to thalamocortical input and distorted sensory encoding. This developmental loss of layer 4 sensory encoding precision would contribute to subsequent developmental alterations in layer 4-to-layer 2/3 connectivity and plasticity observed in Fmr1-KO mice, and circuit dysfunction underlying sensory hypersensitivity. A causal link exists between sensory dysfunction and social and repetitive behaviours in a mouse model of autism (Domanski et al., 2019).

Healthy hippocampal neurons (so-called place cells) exhibit place-related activity during spatial exploration, and their firing fields tend to remain stable over time. Arbab et al., have found impaired stability and reduced specificity of Fmr1-KO spatial representations, which constitutes a potential biomarker for the cognitive dysfunction observed in FXS, informative on the ability to integrate sensory information into an abstract representation and successfully retain this

conceptual memory. Impaired specificity and stability of CA1 place cell activity in Fmr1-KO mice was found, both within and across subsequent exploration sessions, while these mice show a relatively spared place field response and their behavior and firing-rate parameters do not significantly differ from WT mice (Arbab et al., 2018).

Analysis of crude synaptoneurosomes of adult Fmr1 KO mice revealed a significant reduction in Ube3a protein. Additionally, a blunted translation of Ube3a in response to mGluR1/5 stimulation was observed. The majority of AS cases arise from deletions or mutations of UBE3A gene located on the chromosome 15q11-13 (Filonova, 2014).

Fmr1 KO mice backcrossed to the FVB strain and WT littermates were used during experiments. TransnetXY Automated Genotyping (www.transnetyx.com/). TRANSNETYX, INC., 8110 Cordova Rd. Suite 119, Cordova, TN 38016, USA was used for genotyping. The animals were pretreated for 14 days. The active ingredients of Sumatriptan and Ergoloid were in a water carrier, whereas oxitriptan was in a methanol carrier.

The mice were housed in plastic cages (35 x 30 x 12 cm), 5 in each. The room temperature (21 ± 2°C),relative humidity (55 ± 5%), a 12-h light-dark cycle (lights on 7 a.m.-7 p.m.) and air exchange (16 times per h) were automatically controlled. The animals had free access to commercial food pellets and water. Testing was conducted during the light phase. Ten mice per treatment group were used for the AGS experiments. Experiments were conducted in line with the requirements of the UK Animals (Scientific Procedures) Act, 1986.

All experiments were conducted with the experimenter blind to genotype and drug treatment. Separate investigators prepared and coded dosing solutions, allocated the mice to the study treatment groups, dosed the animals, and collected the Audiogenic Seizure data.

*Behavioral Analysis*

Behavior testing was conducted at 2 weeks. The behavioral tests were as follows: 1. Hyperactivity: Open field; 2. Stereotypy: Self-grooming; 3. Sociability: Three chamber partition test; 4. Memory and Learning: Novel Object Recognition; 5. Anxiety: hyponeophagia; and 6. Test of daily living: marble burying

For hyperactivity, the open field test (OFT) is a common measure of exploratory behavior and general activity in both mice and rats, where both the quality and quantity of the activity can be measured. Principally, the open field (OF) is an enclosure, generally square, rectangular, or circular in shape with surrounding walls that prevent escape. The OFT is also commonly used as a mechanism to assess the sedative, toxic, or stimulant effects of compounds (Gould 2009).

For sociability a three chamber partition test was utilized. The three-chamber paradigm test known as Crawley's sociability and preference for social novelty protocol has previously been successfully employed to study social affiliation and social memory in several inbred and mutant mouse lines. The main principle of the test was based on the free choice by a subject mouse to spend time in any of three box's compartments during two experimental sessions, including indirect contact with one or two mice with which it was unfamiliar (Kaidanovich-Beilin 2011).

For memory and learning, a novel object recognition (NOR) task was used to evaluate the rodents' ability to recognize a novel object in the environment. In the NOR task, there are no positive or negative reinforcers, and this methodology assesses the natural preference for novel objects displayed by rodents. The task procedure consists of three phases: habituation, familiarization, and test phase (Antunes 2012).

For anxiety a hyponeophagia test was conducted. Mice and rats cannot vomit, due to the tightness of the cardiac sphincter of the stomach, so to overcome the problem of potential food toxicity they have evolved a strategy of first ingesting only very small amounts of novel substances. The amounts ingested then gradually increase until the animal has determined whether the substance is safe and nutritious. So the old ratcatchers would first put a palatable substance such as oatmeal, which was to be the vehicle for the toxin, in the infested area (Deacon 2011).

For stereotypy, self-grooming was assessed. Self-grooming in animals is an innate behaviour that is involved in hygiene maintenance and other physiologically important processes, including thermoregulation, social communication and de-arousal. It is one of the most frequently observed behaviours in awake rodents and has a patterned, sequential organization with characteristic cephalocaudal progression (Kalueff 2016).

For test of daily living, nesting was assessed as nest building is an innate behavior in rodents, even when raised in laboratory settings. Synthetic and/or natural materials (such as twine, tissue, cotton, paper, and hay) are provided as a gauge of their overall well-being and as an ancillary assessment to predict the possible decline in cognition. Typically, changes in nesting behaviors, such as failure to create a nest, indicate a change in health or welfare. In addition, nesting behavior is sensitive to many environmental and physiological challenges, as well as many genetic mutations underlying pathological disease states (Gaskill 2013).

There are equivalences in human and rodent behavior which can allow animal models to be used to translate how a pharmaceutically active ingredient would be effective in treating human conditions. Some equivalences are as follows:

Social interaction: Poor eye contact, patient prefers to be alone. Development Quotient (DQ)/ Intelligence Quotient (IQ)/ Social Quotient (SQ) according to the Stanford Binet Intelligence Scale or Vineland Social Maturity Scale. (IQ border line intelligence: 71-89)

Problems in communication and/or language: Speech delay, patient pretends to be deaf. Hearing assessment using Brainstem Evoked Response Audiometry (BERA)

Repetitive behavior and/or apparent obsessions: stereotyped behaviour, extreme restlessness and/or hyperactivity. Connor's scale is used to evaluate hyperactivity: >12

The term "disruptive behaviour" has its normal meaning in the art. It may also include repetitive behaviour. It may also include fluctuating mood, irritability, self-injury and aggression.

The term "memory loss" has its normal meaning in the art. It refers to an inability to retain information either short-term or long-term. It may also be called memory impairment. It may include difficulties with cognitive, executive and language performance, executive function and visual memory. It may also include difficulties with working memory, also called short-term memory (i.e. the temporary storage of information while processing the same or other information) and difficulties with phonological memory (or verbal working memory).

The term "social anxiety" has its normal meaning in the art. It may also be termed as difficulties in social interaction or low sociability. Social anxiety may include having poor eye contact, gaze aversion, prolonged time to commence social interaction, social avoidance or withdrawal and challenges forming peer relationships.

The term "hyperactivity" has its normal meaning in the art. Hyperactivity may include having very short attention spans, hypersensitivity to visual, auditory, tactile, and olfactory stimuli, distractibility, impulsiveness, restlessness and/or over-activity.

*Treatment Regime*

The treatment of the mice with OX (Oxitriptan), SU (Sumatriptan), ER (Ergoloid mesylates, also referred to as ergoloid herein) were according to the matrix shown below in Table 1.

Table 1

| Group Number | Dosing route | Dose | Dosing Regiment | No. animals |
|---|---|---|---|---|
| 1 WT | IP | N/A | QD | 10 |
| 2 KO | IP | N/A | QD | 10 |
| 3 KO OX | IP | 80 mg/kg | QD | 10 |
| 4 KO SU | IP | 20 mg/kg | QD | 10 |
| 5 KO ER | PO | 4 mg/kg | QD | 10 |
| 6 KO ER + OX | PO + IP | 2 mg/kg + 40 mg/kg | QD | 10 |

*Results*

The results of the behavioral tests of the mice are provided in Tables 2 to 12 below.

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 6 | | | | | | | |
| Alpha | 0.05 | | | | | | | |
| | | | | | | | | |
| **Dunnett's multiple comparisons test** | **Mean Diff.** | **95.00% CI of diff.** | **Below threshold?** | **Summary** | **Adjusted P Value** | **A-?** | | |
| WT-V vs. KO-V | -4055 | -4459 to -3651 | Yes | **** | <0.0001 | B | KO-V | |
| WT-V vs. KO-Sumatriptan (20 mg/kg) | -3209 | -3612 to -2805 | Yes | **** | <0.0001 | C | KO-Sumatriptan (20 mg/kg) | |
| WT-V vs. KO - 5-HTP (80 mg/kg) | -512.4 | -916.2 to -108.6 | Yes | ** | 0.0073 | D | KO - 5-HTP (80 mg/kg) | |
| WT-V vs. KO-EM (4 mg/kg) | -3076 | -3480 to -2672 | Yes | **** | <0.0001 | E | KO-EM (4 mg/kg) | |
| WT-V vs. Column F | -4009 | -4412 to -3605 | Yes | **** | <0.0001 | F | Column F | |
| WT-V vs. Column G | 20.00 | -383.8 to 423.8 | No | ns | 0.9998 | G | Column G | |
| | | | | | | | | |
| **Test details** | **Mean 1** | **Mean 2** | **Mean Diff.** | **SE of diff.** | **n1** | **n2** | **q** | **DF** |
| WT-V vs. KO-V | 4207 | 8263 | -4055 | 153.1 | 10 | 10 | 26.49 | 63 |
| WT-V vs. KO-Sumatriptan (20 mg/kg) | 4207 | 7416 | -3209 | 153.1 | 10 | 10 | 20.96 | 63 |
| WT-V vs. KO - 5-HTP (80 mg/kg) | 4207 | 4720 | -512.4 | 153.1 | 10 | 10 | 3.348 | 63 |
| WT-V vs. KO-EM (4 mg/kg) | 4207 | 7284 | -3076 | 153.1 | 10 | 10 | 20.10 | 63 |
| WT-V vs. Column F | 4207 | 8216 | -4009 | 153.1 | 10 | 10 | 26.19 | 63 |
| WT-V vs. Column G | 4207 | 4187 | 20.00 | 153.1 | 10 | 10 | 0.1307 | 63 |

Table 2 One-way analysis of variance and multiple comparisons analysis against WT-V (Open Field). Column F corresponds to the suggested KO- Ergoloid (2mg/kg) and Sumatriptan (10 mg/kg). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 6 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **A-?** | | |
| WT-V vs. KO-V | -91.80 | -99.29 to -84.31 | Yes | **** | <0.0001 | B | KO-V | |
| WT-V vs. KO-Sumatriptan (20 mg/kg) | -9.200 | -16.69 to -1.710 | Yes | ** | 0.0100 | C | KO-Sumatriptan (20 mg/kg) | |
| WT-V vs. KO-Oxitriptan (40 mg/kg) | -0.7000 | -8.190 to 6.790 | No | ns | 0.9996 | D | KO-Oxitriptan (40 mg/kg) | |
| WT-V vs. KO-Ergoloid (4 mg/kg) | -0.7000 | -8.190 to 6.790 | No | ns | 0.9996 | E | KO-Ergoloid (4 mg/kg) | |
| WT-V vs. Column F | -92.30 | -99.79 to -84.81 | Yes | **** | <0.0001 | F | Column F | |
| WT-V vs. Column G | -0.7000 | -8.190 to 6.790 | No | ns | 0.9996 | G | Column G | |
| | | | | | | | | |
| **Test details** | **Mean 1** | **Mean 2** | **Mean Diff.** | **SE of diff.** | **n1** | **n2** | **q** | **DF** |
| WT-V vs. KO-V | 145.5 | 237.3 | -91.80 | 2.839 | 10 | 10 | 32.33 | 63 |
| WT-V vs. KO-Sumatriptan (20 mg/kg) | 145.5 | 154.7 | -9.200 | 2.839 | 10 | 10 | 3.240 | 63 |
| WT-V vs. KO-Oxitriptan (40 mg/kg) | 145.5 | 146.2 | -0.7000 | 2.839 | 10 | 10 | 0.2466 | 63 |
| WT-V vs. KO-Ergoloid (4 mg/kg) | 145.5 | 146.2 | -0.7000 | 2.839 | 10 | 10 | 0.2466 | 63 |
| WT-V vs. Column F | 145.5 | 237.8 | -92.30 | 2.839 | 10 | 10 | 32.51 | 63 |
| WT-V vs. Column G | 145.5 | 146.2 | -0.7000 | 2.839 | 10 | 10 | 0.2466 | 63 |

Table 3. One-way analysis of variance and multiple comparisons analysis against WT-V (Hyponeophagia). Column F corresponds to the suggested KO- Ergoloid (2mg/kg) and Sumatriptan (10 mg/kg). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 6 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **A-?** | | |
| WT-V vs. KO-V | 3.600 | 3.096 to 4.104 | Yes | **** | <0.0001 | B | KO-V | |
| WT-V vs. KO-Sumatriptan (20 mg/kg) | 0.1000 | -0.4041 to 0.6041 | No | ns | 0.9896 | C | KO-Sumatriptan (20 mg/kg) | |
| WT-V vs. KO-Oxitriptan (40 mg/kg) | -0.1000 | -0.6041 to 0.4041 | No | ns | 0.9896 | D | KO-Oxitriptan (40 mg/kg) | |
| WT-V vs. KO-Ergoloid (4 mg/kg) | 0.000 | -0.5041 to 0.5041 | No | ns | >0.9999 | E | KO-Ergoloid (4 mg/kg) | |
| WT-V vs. Column F | 1.500 | 0.9959 to 2.004 | Yes | **** | <0.0001 | F | Column F | |
| WT-V vs. Column G | 0.000 | -0.5041 to 0.5041 | No | ns | >0.9999 | G | Column G | |
| | | | | | | | | |
| **Test details** | **Mean 1** | **Mean 2** | **Mean Diff.** | **SE of diff.** | **n1** | **n2** | **q** | **DF** |
| WT-V vs. KO-V | 4.800 | 1.200 | 3.600 | 0.1911 | 10 | 10 | 18.84 | 63 |
| WT-V vs. KO-Sumatriptan (20 mg/kg) | 4.800 | 4.700 | 0.1000 | 0.1911 | 10 | 10 | 0.5234 | 63 |
| WT-V vs. KO-Oxitriptan (40 mg/kg) | 4.800 | 4.900 | -0.1000 | 0.1911 | 10 | 10 | 0.5234 | 63 |
| WT-V vs. KO-Ergoloid (4 mg/kg) | 4.800 | 4.800 | 0.000 | 0.1911 | 10 | 10 | 0.000 | 63 |
| WT-V vs. Column F | 4.800 | 3.300 | 1.500 | 0.1911 | 10 | 10 | 7.851 | 63 |
| WT-V vs. Column G | 4.800 | 4.800 | 0.000 | 0.1911 | 10 | 10 | 0.000 | 63 |

Table 4. One-way analysis of variance and multiple comparisons analysis against WT-V (Test of daily living). Column F corresponds to the suggested KO- Ergoloid (2mg/kg) and Sumatriptan (10 mg/kg). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 6 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **B-?** | | |
| N_WT-V vs. N_KO-V | 7.200 | 5.125 to 9.275 | Yes | **** | <0.0001 | D | N_KO-V | |
| N_WT-V vs. N_KO-Sumatriptan (20 mg/kg) | 6.600 | 4.525 to 8.675 | Yes | **** | <0.0001 | F | N_KO-Sumatriptan (20 mg/kg) | |
| N_WT-V vs. N_KO-Oxitriptan (40 mg/kg) | 3.082 | 1.055 to 5.109 | Yes | *** | 0.0009 | H | N_KO-Oxitriptan (40 mg/kg) | |
| N_WT-V vs. N_KO-Ergoloid (4 mg/kg) | 2.900 | 0.8730 to 4.927 | Yes | ** | 0.0020 | J | N_KO-Ergoloid (4 mg/kg) | |
| N_WT-V vs. Column L | 6.900 | 4.873 to 8.927 | Yes | **** | <0.0001 | L | Column L | |
| N_WT-V vs. Column N | -0.6000 | -2.675 to 1.475 | No | ns | 0.9353 | N | Column N | |
| | | | | | | | | |
| **Test details** | **Mean 1** | **Mean 2** | **Mean Diff.** | **SE of diff.** | **n1** | **n2** | **q** | **DF** |
| N_WT-V vs. N_KO-V | 13.90 | 6.700 | 7.200 | 0.7886 | 10 | 10 | 9.130 | 66 |
| N_WT-V vs. N_KO-Sumatriptan (20 mg/kg) | 13.90 | 7.300 | 6.600 | 0.7886 | 10 | 10 | 8.369 | 66 |
| N_WT-V vs. N_KO-Oxitriptan (40 mg/kg) | 13.90 | 10.82 | 3.082 | 0.7705 | 10 | 11 | 4.000 | 66 |
| N_WT-V vs. N_KO-Ergoloid (4 mg/kg) | 13.90 | 11.00 | 2.900 | 0.7705 | 10 | 11 | 3.764 | 66 |
| N_WT-V vs. Column L | 13.90 | 7.000 | 6.900 | 0.7705 | 10 | 11 | 8.955 | 66 |
| N_WT-V vs. Column N | 13.90 | 14.50 | -0.6000 | 0.7886 | 10 | 10 | 0.7608 | 66 |

Table 5. One-way analysis of variance and multiple comparisons analysis against WT-V (NOR). Column L corresponds to F_KO-Ergoloid (2mg/kg) and Sumatriptan (10 mg/kg) and column N to N_KO- Ergoloid (2mg/kg) and Oxitriptan (40 mg/kg). Column N corresponds to F_KO- Ergoloid (2mg/kg) and Oxitriptan (40 mg/kg) and column N to N_KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 6 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **B-?** | | |
| N_WT-V vs. N_KO-V | 15.20 | 11.87 to 18.53 | Yes | **** | <0.0001 | D | N_KO-V | |
| N_WT-V vs. N_KO-Sumatriptan (20 mg/kg) | 0.9000 | -2.429 to 4.229 | No | ns | 0.9529 | F | N_KO-Sumatriptan (20 mg/kg) | |
| N_WT-V vs. N_KO-Oxitriptan (40 mg/kg) | 0.3000 | -3.029 to 3.629 | No | ns | 0.9997 | H | N_KO-Oxitriptan (40 mg/kg) | |
| N_WT-V vs. N_KO-Ergoloid (4 mg/kg) | -1.300 | -4.629 to 2.029 | No | ns | 0.8027 | J | N_KO-Ergoloid (4 mg/kg) | |
| N_WT-V vs. Column L | 15.20 | 11.87 to 18.53 | Yes | **** | <0.0001 | L | Column L | |
| N_WT-V vs. Column N | -0.4556 | -3.875 to 2.964 | No | ns | 0.9981 | N | Column N | |
| | | | | | | | | |
| **Test details** | **Mean 1** | **Mean 2** | **Mean Diff.** | **SE of diff.** | **n1** | **n2** | **q** | **DF** |
| N_WT-V vs. N_KO-V | 99.10 | 83.90 | 15.20 | 1.260 | 10 | 10 | 12.06 | 62 |
| N_WT-V vs. N_KO-Sumatriptan (20 mg/kg) | 99.10 | 98.20 | 0.9000 | 1.260 | 10 | 10 | 0.7140 | 62 |
| N_WT-V vs. N_KO-Oxitriptan (40 mg/kg) | 99.10 | 98.80 | 0.3000 | 1.260 | 10 | 10 | 0.2380 | 62 |
| N_WT-V vs. N_KO-Ergoloid (4 mg/kg) | 99.10 | 100.4 | -1.300 | 1.260 | 10 | 10 | 1.031 | 62 |
| N_WT-V vs. Column L | 99.10 | 83.90 | 15.20 | 1.260 | 10 | 10 | 12.06 | 62 |
| N_WT-V vs. Column N | 99.10 | 99.56 | -0.4556 | 1.295 | 10 | 9 | 0.3518 | 62 |

Table 6. One-way analysis of variance and multiple comparisons analysis against WT-V (Sociability). Column L corresponds to F_KO-Ergoloid (2mg/kg) and Sumatriptan (10 mg/kg) and column N to N_KO- Ergoloid (2mg/kg) and Oxitriptan (40 mg/kg). Column N corresponds to F_KO- Ergoloid (2mg/kg) and Oxitriptan (40 mg/kg) and column N to N_KO- Ergoloid (2mg/kg) and Oxitriptan (40 mg/kg).

| Ordinary one-way ANOVA Multiple comparisons | | | | | | | | |
|---|---|---|---|---|---|---|---|---|
| Number of families | 1 | | | | | | | |
| Number of comparisons per family | 5 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **B-?** | | |
| KO-V vs. WT-V | 4055 | 3659 to 4451 | Yes | **** | <0.0001 | A | WT-V | |
| KO-V vs. KO-Sumatriptan (20 mg/kg) | 846.6 | 450.8 to 1242 | Yes | **** | <0.0001 | C | KO-Sumatriptan (20 mg/kg) | |
| KO-V vs. KO-Oxitriptan (80 mg/kg) | 3543 | 3147 to 3939 | Yes | **** | <0.0001 | D | KO-Oxitriptan (80 mg/kg) | |
| KO-V vs. KO-Ergoloid (4 mg/kg) | 979.0 | 583.2 to 1375 | Yes | **** | <0.0001 | E | KO-Ergoloid (4 mg/kg) | |
| KO-V vs. Column G | 4075 | 3679 to 4471 | Yes | **** | <0.0001 | G | Column G | |
| | | | | | | | | |
| **Test details** | **Mean 1** | **Mean 2** | **Mean Diff.** | **SE of diff.** | **n1** | **n2** | **q** | **DF** |
| KO-V vs. WT-V | 8263 | 4207 | 4055 | 152.8 | 10 | 10 | 26.54 | 54 |
| KO-V vs. KO-Sumatriptan (20 mg/kg) | 8263 | 7416 | 846.6 | 152.8 | 10 | 10 | 5.542 | 54 |
| KO-V vs. KO-Oxitriptan (80 mg/kg) | 8263 | 4720 | 3543 | 152.8 | 10 | 10 | 23.19 | 54 |
| KO-V vs. KO-Ergoloid (4 mg/kg) | 8263 | 7284 | 979.0 | 152.8 | 10 | 10 | 6.408 | 54 |
| KO-V vs. Column G | 8263 | 4187 | 4075 | 152.8 | 10 | 10 | 26.67 | 54 |

Table 7. One-way analysis of variance and multiple comparisons analysis against KO-V (Open Field). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 5 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **B-?** | | |
| KO-V vs. WT-V | 13.80 | 10.57 to 17.03 | Yes | **** | <0.0001 | A | WT-V | |
| KO-V vs. KO-Sumatriptan (20 mg/kg) | 14.50 | 11.27 to 17.73 | Yes | **** | <0.0001 | C | KO-Sumatriptan (20 mg/kg) | |
| KO-V vs. KO-Oxitriptan (40 mg/kg) | 10.10 | 6.868 to 13.33 | Yes | **** | <0.0001 | D | KO-Oxitriptan (40 mg/kg) | |
| KO-V vs. KO-Ergoloid (4 mg/kg) | 12.20 | 8.968 to 15.43 | Yes | **** | <0.0001 | E | KO-Ergoloid (4 mg/kg) | |
| KO-V vs. Column G | 14.50 | 11.27 to 17.73 | Yes | **** | <0.0001 | G | Column G | |
| | | | | | | | | |
| **Test details** | **Mean 1** | **Mean 2** | **Mean Diff.** | **SE of diff.** | **n1** | **n2** | **q** | **DF** |
| KO-V vs. WT-V | 45.40 | 31.60 | 13.80 | 1.248 | 10 | 10 | 11.06 | 54 |
| KO-V vs. KO-Sumatriptan (20 mg/kg) | 45.40 | 30.90 | 14.50 | 1.248 | 10 | 10 | 11.62 | 54 |
| KO-V vs. KO-Oxitriptan (40 mg/kg) | 45.40 | 35.30 | 10.10 | 1.248 | 10 | 10 | 8.095 | 54 |
| KO-V vs. KO-Ergoloid (4 mg/kg) | 45.40 | 33.20 | 12.20 | 1.248 | 10 | 10 | 9.778 | 54 |
| KO-V vs. Column G | 45.40 | 30.90 | 14.50 | 1.248 | 10 | 10 | 11.62 | 54 |

Table 8. One-way analysis of variance and multiple comparisons analysis against KO-V (Stereotypy). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 5 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **B-?** | | |
| KO-V vs. WT-V | 91.80 | 85.31 to 98.29 | Yes | **** | <0.0001 | A | WT-V | |
| KO-V vs. KO-Sumatriptan (20 mg/kg) | 82.60 | 76.11 to 89.09 | Yes | **** | <0.0001 | C | KO-Sumatriptan (20 mg/kg) | |
| KO-V vs. KO-Oxitriptan (40 mg/kg) | 91.10 | 84.61 to 97.59 | Yes | **** | <0.0001 | D | KO-Oxitriptan (40 mg/kg) | |
| KO-V vs. KO-Ergoloid (4 mg/kg) | 91.10 | 84.61 to 97.59 | Yes | **** | <0.0001 | E | KO-Ergoloid (4 mg/kg) | |
| KO-V vs. Column G | 91.10 | 84.61 to 97.59 | Yes | **** | <0.0001 | G | Column G | |
| | | | | | | | | |
| **Test details** | **Mean 1** | **Mean 2** | **Mean Diff.** | **SE of diff.** | **n1** | **n2** | **q** | **DF** |
| KO-V vs. WT-V | 237.3 | 145.5 | 91.80 | 2.504 | 10 | 10 | 36.65 | 54 |
| KO-V vs. KO-Sumatriptan (20 mg/kg) | 237.3 | 154.7 | 82.60 | 2.504 | 10 | 10 | 32.98 | 54 |
| KO-V vs. KO-Oxitriptan (40 mg/kg) | 237.3 | 146.2 | 91.10 | 2.504 | 10 | 10 | 36.38 | 54 |
| KO-V vs. KO-Ergoloid (4 mg/kg) | 237.3 | 146.2 | 91.10 | 2.504 | 10 | 10 | 36.38 | 54 |
| KO-V vs. Column G | 237.3 | 146.2 | 91.10 | 2.504 | 10 | 10 | 36.38 | 54 |

Table 9. One-way analysis of variance and multiple comparisons analysis against KO-V (Hyponeophagia). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 5 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **B-?** | | |
| KO-V vs. WT-V | -3.600 | -4.083 to -3.117 | Yes | **** | <0.0001 | A | WT-V | |
| KO-V vs. KO-Sumatriptan (20 mg/kg) | -3.500 | -3.983 to -3.017 | Yes | **** | <0.0001 | C | KO-Sumatriptan (20 mg/kg) | |
| KO-V vs. KO-Oxitriptan (40 mg/kg) | -3.700 | -4.183 to -3.217 | Yes | **** | <0.0001 | D | KO-Oxitriptan (40 mg/kg) | |
| KO-V vs. KO-Ergoloid (4 mg/kg) | -3.600 | -4.083 to -3.117 | Yes | **** | <0.0001 | E | KO-Ergoloid (4 mg/kg) | |
| KO-V vs. Column G | -3.600 | -4.083 to -3.117 | Yes | **** | <0.0001 | G | Column G | |
| | | | | | | | | |
| **Test details** | **Mean 1** | **Mean 2** | **Mean Diff.** | **SE of diff.** | **n1** | **n2** | **q** | **DF** |
| KO-V vs. WT-V | 1.200 | 4.800 | -3.600 | 0.1866 | 10 | 10 | 19.29 | 54 |
| KO-V vs. KO-Sumatriptan (20 mg/kg) | 1.200 | 4.700 | -3.500 | 0.1866 | 10 | 10 | 18.76 | 54 |
| KO-V vs. KO-Oxitriptan (40 mg/kg) | 1.200 | 4.900 | -3.700 | 0.1866 | 10 | 10 | 19.83 | 54 |
| KO-V vs. KO-Ergoloid (4 mg/kg) | 1.200 | 4.800 | -3.600 | 0.1866 | 10 | 10 | 19.29 | 54 |
| KO-V vs. Column G | 1.200 | 4.800 | -3.600 | 0.1866 | 10 | 10 | 19.29 | 54 |

Table 10. One-way analysis of variance and multiple comparisons analysis against KO-V (Test of daily living). Column G corresponds to the suggested KO- Ergoloid (2mg/kg) and Oxitriptan (40 mg/kg).

| Ordinary one-way ANOVA Multiple comparisons | | | | | | | | |
|---|---|---|---|---|---|---|---|---|
| | | | | | | | | |
| Number of families | 1 | | | | | | | |
| Number of comparisons per family | 11 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **C-?** | | |
| F_KO-V vs. F_WT-V | 1.500 | -0.5775 to 3.578 | No | ns | 0.2810 | A | F_WT-V | |
| F_KO-V vs. N_WT-V | -7.300 | -9.378 to -5.222 | Yes | **** | <0.0001 | B | N_WT-V | |
| F_KO-V vs. N_KO-V | -0.1000 | -2.178 to 1.978 | No | ns | 0.9998 | D | N_KO-V | |
| F_KO-V vs. F_KO-Sumatriptan (20 mg/kg) | -0.2000 | -2.278 to 1.878 | No | ns | 0.9996 | E | F_KO-Sumatriptan (20 mg/kg) | |
| F_KO-V vs. N_KO-Sumatriptan (20 mg/kg) | -0.7000 | -2.778 to 1.378 | No | ns | 0.9561 | F | N_KO-Sumatriptan (20 mg/kg) | |
| F_KO-V vs. F_KO-Oxitriptan (40 mg/kg) | -0.03636 | -2.066 to 1.993 | No | ns | >0.9999 | G | F_KO-Oxitriptan (40 mg/kg) | |
| F_KO-V vs. N_KO-Oxitriptan (40 mg/kg) | -4.218 | -6.248 to -2.188 | Yes | **** | <0.0001 | H | N_KO-Oxitriptan (40 mg/kg) | |
| F_KO-V vs. F_KO-Ergoloid (4 mg/kg) | 0.1455 | -1.884 to 2.175 | No | ns | 0.9997 | I | F_KO-Ergoloid (4 mg/kg) | |
| F_KO-V vs. N_KO-Ergoloid (4 mg/kg) | -4.400 | -6.430 to -2.370 | Yes | **** | <0.0001 | J | N_KO-Ergoloid (4 mg/kg) | |
| F_KO-V vs. Column M | 1.300 | -0.7775 to 3.378 | No | ns | 0.4426 | M | Column M | |
| F_KO-V vs. Column N | -7.900 | -9.978 to -5.822 | Yes | **** | <0.0001 | N | Column N | |
| | | | | | | | | |
| **Test details** | **Mean 1** | **Mean 2** | **Mean Diff.** | **SE of diff.** | **n1** | **n2** | **q** | **DF** |
| F_KO-V vs. F_WT-V | 6.600 | 5.100 | 1.500 | 0.7458 | 10 | 10 | 2.011 | 112 |
| F_KO-V vs. N_WT-V | 6.600 | 13.90 | -7.300 | 0.7458 | 10 | 10 | 9.788 | 112 |
| F_KO-V vs. N_KO-V | 6.600 | 6.700 | -0.1000 | 0.7458 | 10 | 10 | 0.1341 | 112 |
| F_KO-V vs. F_KO-Sumatriptan (20 mg/kg) | 6.600 | 6.800 | -0.2000 | 0.7458 | 10 | 10 | 0.2682 | 112 |
| F_KO-V vs. N_KO-Sumatriptan (20 mg/kg) | 6.600 | 7.300 | -0.7000 | 0.7458 | 10 | 10 | 0.9385 | 112 |
| F_KO-V vs. F_KO-Oxitriptan (40 mg/kg) | 6.600 | 6.636 | -0.03636 | 0.7287 | 10 | 11 | 0.04990 | 112 |
| F_KO-V vs. N_KO-Oxitriptan (40 mg/kg) | 6.600 | 10.82 | -4.218 | 0.7287 | 10 | 11 | 5.789 | 112 |
| F_KO-V vs. F_KO-Ergoloid (4 mg/kg) | 6.600 | 6.455 | 0.1455 | 0.7287 | 10 | 11 | 0.1996 | 112 |
| F_KO-V vs. N_KO-Ergoloid (4 mg/kg) | 6.600 | 11.00 | -4.400 | 0.7287 | 10 | 11 | 6.038 | 112 |
| F_KO-V vs. Column M | 6.600 | 5.300 | 1.300 | 0.7458 | 10 | 10 | 1.743 | 112 |
| F_KO-V vs. Column N | 6.600 | 14.50 | -7.900 | 0.7458 | 10 | 10 | 10.59 | 112 |

Table 11. One-way analysis of variance and multiple comparisons analysis against F_KO-V (NOR). Column M corresponds to F_KO-Ergoloid (2mg/kg) and Oxitriptan (40 mg/kg) and column N to N_KO- Ergoloid (2mg/kg) and Oxitriptan (40 mg/kg).

| Ordinary one-way ANOVA<br>Multiple comparisons | | | | | | | | |
|---|---|---|---|---|---|---|---|---|
| | | | | | | | | |
| Number of families | 1 | | | | | | | |
| Number of comparisons per family | 11 | | | | | | | |
| Alpha | 0.05 | | | | | | | |
| | | | | | | | | |
| **Dunnett's multiple comparisons test** | **Mean Diff.** | **95.00% CI of diff.** | **Significant?** | **Summary** | **Adjusted P Value** | **C-?** | | |
| F_KO-V vs. F_WT-V | 29.30 | 26.21 to 32.39 | Yes | **** | <0.0001 | A | F_WT-V | |
| F_KO-V vs. N_WT-V | -17.50 | -20.59 to -14.41 | Yes | **** | <0.0001 | B | N_WT-V | |
| F_KO-V vs. N_KO-V | -2.300 | -5.390 to 0.7905 | No | ns | 0.2520 | D | N_KO-V | |
| F_KO-V vs. F_KO-Sumatriptan (20 mg/kg) | 19.40 | 16.31 to 22.49 | Yes | **** | <0.0001 | E | F_KO-Sumatriptan (20 mg/kg) | |
| F_KO-V vs. N_KO-Sumatriptan (20 mg/kg) | -16.60 | -19.69 to -13.51 | Yes | **** | <0.0001 | F | N_KO-Sumatriptan (20 mg/kg) | |
| F_KO-V vs. F_KO-Oxitriptan (40 mg/kg) | 29.50 | 26.41 to 32.59 | Yes | **** | <0.0001 | G | F_KO-Oxitriptan (40 mg/kg) | |
| F_KO-V vs. N_KO-Oxitriptan (40 mg/kg) | -17.20 | -20.29 to -14.11 | Yes | **** | <0.0001 | H | N_KO-Oxitriptan (40 mg/kg) | |
| F_KO-V vs. F_KO-Ergoloid (4 mg/kg) | 29.40 | 26.31 to 32.49 | Yes | **** | <0.0001 | I | F_KO-Ergoloid (4 mg/kg) | |
| F_KO-V vs. N_KO-Ergoloid (4 mg/kg) | -18.80 | -21.89 to -15.71 | Yes | **** | <0.0001 | J | N_KO-Ergoloid (4 mg/kg) | |
| F_KO-V vs. Column M | 29.16 | 25.98 to 32.33 | Yes | **** | <0.0001 | M | Column M | |
| F_KO-V vs. Column N | -17.96 | -21.13 to -14.78 | Yes | **** | <0.0001 | N | Column N | |
| | | | | | | | | |
| **Test details** | **Mean 1** | **Mean 2** | **Mean Diff.** | **SE of diff.** | **n1** | **n2** | **q** | **DF** |
| F_KO-V vs. F_WT-V | 81.60 | 52.30 | 29.30 | 1.106 | 10 | 10 | 26.49 | 106 |
| F_KO-V vs. N_WT-V | 81.60 | 99.10 | -17.50 | 1.106 | 10 | 10 | 15.82 | 106 |
| F_KO-V vs. N_KO-V | 81.60 | 83.90 | -2.300 | 1.106 | 10 | 10 | 2.079 | 106 |
| F_KO-V vs. F_KO-Sumatriptan (20 mg/kg) | 81.60 | 62.20 | 19.40 | 1.106 | 10 | 10 | 17.54 | 106 |
| F_KO-V vs. N_KO-Sumatriptan (20 mg/kg) | 81.60 | 98.20 | -16.60 | 1.106 | 10 | 10 | 15.01 | 106 |
| F_KO-V vs. F_KO-Oxitriptan (40 mg/kg) | 81.60 | 52.10 | 29.50 | 1.106 | 10 | 10 | 26.67 | 106 |
| F_KO-V vs. N_KO-Oxitriptan (40 mg/kg) | 81.60 | 98.80 | -17.20 | 1.106 | 10 | 10 | 15.55 | 106 |
| F_KO-V vs. F_KO-Ergoloid (4 mg/kg) | 81.60 | 52.20 | 29.40 | 1.106 | 10 | 10 | 26.58 | 106 |
| F_KO-V vs. N_KO-Ergoloid (4 mg/kg) | 81.60 | 100.4 | -18.80 | 1.106 | 10 | 10 | 17.00 | 106 |
| F_KO-V vs. Column M | 81.60 | 52.44 | 29.16 | 1.136 | 10 | 9 | 25.66 | 106 |
| F_KO-V vs. Column N | 81.60 | 99.56 | -17.96 | 1.136 | 10 | 9 | 15.80 | 106 |

Table 12. One-way analysis of variance and multiple comparisons analysis against F_KO-V (Sociability). Column M corresponds to F_KO- Ergoloid (2mg/kg) and Oxitriptan (40 mg/kg) and column N to N_KO- Ergoloid (2mg/kg) and Oxitriptan (40 mg/kg).

Detailed results of the behavioral mice experiments are detailed below in Tables 13 to 20.

Table 13. Data table Open field

| WT-V | KO-V | KO-Sumatrip | KO-Oxitripta | KO-Ergoloid (4 mg/kg) | | KO - Ergoloid (2 mg/kg) + Oxitriptan (40 mg/kg) |
|---|---|---|---|---|---|---|
| 3825 | 7844 | 7073 | 4835 | 6125 | | 4016 |
| 4061 | 8194 | 7945 | 4592 | 7937 | | 4238 |
| 4023 | 7905 | 8168 | 4317 | 7318 | | 4404 |
| 4502 | 8038 | 7042 | 4635 | 7547 | | 4133 |
| 4438 | 8510 | 7083 | 4981 | 7831 | | 4409 |
| 4197 | 9153 | 7418 | 4732 | 7390 | | 4238 |
| 4611 | 8532 | 7290 | 4891 | 7244 | | 4391 |
| 4193 | 8045 | 7735 | 4936 | 7018 | | 3805 |
| 4038 | 8342 | 7244 | 4771 | 7283 | | 4122 |
| 4186 | 8063 | 7162 | 4508 | 7143 | | 4118 |

Table 14. Data table Stereotypy

| WT-V | KO-V | KO-Sumatriptan (20 mg/kg) | KO-Oxitriptan (40 mg/kg) | KO-Ergoloid (4 mg/kg) | KO - Ergoloid (2 mg/kg) + Oxitriptan (40 mg/kg) |
|---|---|---|---|---|---|
| 27 | 50 | 30 | 29 | 32 | 30 |
| 33 | 47 | 29 | 34 | 35 | 29 |
| 30 | 44 | 35 | 37 | 33 | 35 |
| 29 | 45 | 31 | 40 | 27 | 31 |
| 33 | 48 | 30 | 37 | **34** | 30 |
| 30 | 50 | 32 | 36 | 32 | 32 |
| 31 | 43 | 28 | 38 | 35 | 28 |
| 35 | 44 | 30 | 33 | 38 | 30 |
| 32 | 40 | 31 | 38 | 32 | 31 |
| 36 | 43 | 33 | 31 | 34 | 33 |

Table 15. Data table Sociability

| F_WT-V | N_WT-V | F_KO-V | N_KO-V | F_KO-Sumatriptan (20 mg/kg) | N_KO-Sumatriptan (20 mg/kg) | F_KO-Oxitriptan (40 mg/kg) | N_KO-Oxitriptan (40 mg/kg) | F_KO-Ergoloid (4 mg/kg) | N_KO-Ergoloid (4 mg/kg) |
|---|---|---|---|---|---|---|---|---|---|
| 55 | 98 | 85 | 88 | 60 | 100 | 50 | 99 | 53 | 102 |
| 50 | 100 | 77 | 83 | 63 | 95 | 53 | 97 | 52 | 100 |
| 53 | 99 | 80 | 80 | 65 | 101 | 55 | 100 | 51 | 99 |
| 51 | 103 | 85 | 84 | 61 | 99 | 51 | 104 | 50 | 104 |
| 52 | 100 | 81 | 87 | 64 | 97 | 54 | 98 | 52 | 99 |
| 54 | 95 | 80 | 85 | 65 | 103 | 52 | 97 | 54 | 97 |
| 50 | 97 | 84 | 81 | 60 | 98 | 50 | 100 | 51 | 101 |
| 52 | 102 | 82 | 83 | 61 | 90 | 52 | 99 | 50 | 100 |
| 51 | 100 | 79 | 80 | 60 | 97 | 53 | 96 | 56 | 98 |
| 55 | 97 | 83 | 88 | 63 | 102 | 51 | 98 | 53 | 104 |

Table 16. Data table Sociability Continued (highlighting combination results)

| F_KO - Ergoloid (2 mg/kg) + Oxitriptan (40 mg/kg) | N_KO - Ergoloid (2 mg/kg) + Oxitriptan (40 mg/kg) |
|---|---|
| 54 | 100 |
| 50 | 98 |
| 57 | 103 |
| 53 | 97 |
| 50 | 99 |
| 52 | 103 |
| 55 | 101 |
| 51 | 97 |
| 50 | 98 |
| | |

Table 17. Data table Novel Object Recognition

| F_WT-V | N_WT-V | E_KO-V | N_KO-V | F_KO-Sumatriptan (20 mg/kg) | N_KO-Sumatriptan (20 mg/kg) | F_KO-Oxitriptan (40 mg/kg) | N_KO-Oxitriptan (40 mg/kg) | F_KO-Ergoloid (4 mg/kg) | N_KO-Ergoloid (4 mg/kg) |
|---|---|---|---|---|---|---|---|---|---|
| 4 | 13 | 9 | 5 | 4 | 7 | 6 | 10 | 8 | 12 |
| 7 | 15 | 5 | 7 | 8 | 6 | 8 | 9 | 5 | 10 |
| 5 | 12 | 7 | 5 | 6 | 9 | 5 | 8 | 7 | 14 |
| 3 | 16 | 8 | 8 | 9 | 6 | 6 | 10 | 9 | 11 |
| 4 | 13 | 6 | 6 | 5 | 8 | 5 | 12 | 6 | 10 |
| 6 | 17 | 5 | 9 | 8 | 9 | 9 | 14 | 5 | 9 |
| 5 | 12 | 5 | 6 | 7 | 5 | 6 | 10 | 8 | 12 |
| 7 | 14 | 7 | 8 | 9 | 8 | 8 | 8 | 4 | 13 |
| 4 | 15 | 6 | 5 | 7 | 6 | 6 | 10 | 6 | 10 |
| 6 | 12 | 8 | 8 | 5 | 9 | 9 | 15 | 5 | 9 |
|  |  |  |  |  |  | 5 | 13 | 8 | 11 |

Table 18. Data table Novel Object Recognition (highlighting combination results)

| F_KO - Ergoloid (2 mg/kg) + Oxitriptan (40 mg/kg) | N_KO - Ergoloid (2 mg/kg) + Oxitriptan (40 mg/kg) |
|---|---|
| 7 | 15 |
| 4 | 17 |
| 5 | 12 |
| 3 | 14 |
| 6 | 12 |
| 7 | 16 |
| 5 | 14 |
| 4 | 17 |
| 6 | 13 |
| 6 | 15 |
| | |

Table 19. Data table Hyponeophagia

| WT-V | KO-V | KO-Sumatriptan (20 mg/kg) | KO-Oxitriptan (40 mg/kg) | KO-Ergoloid (4 mg/kg) | KO - Ergoloid (2 mg/kg) + Oxitriptan (40 mg/kg) |
|---|---|---|---|---|---|
| 145 | 230 | 152 | 146 | 142 | 146 |
| 140 | 222 | 154 | 151 | 139 | 151 |
| 138 | 250 | 157 | 145 | 152 | 145 |
| 153 | 233 | 148 | 150 | 155 | 150 |
| 150 | 247 | 153 | 140 | 149 | 140 |
| 144 | 240 | 155 | 147 | 143 | 147 |
| 146 | 239 | 158 | 143 | 141 | 143 |
| 148 | 241 | 154 | 140 | 153 | 140 |
| 141 | 228 | 157 | 152 | 150 | 152 |
| 150 | 243 | 159 | 148 | 138 | 148 |

Table 20. Data table Tests of daily living

| WT-V | KO-V | KO-Sumatriptan (20 mg/kg) | KO-Oxitriptan (40 mg/kg) | KO-Ergoloid (4 mg/kg) | KO - Ergoloid (2 mg/kg) + Oxitriptan (40 mg/kg) |
|---|---|---|---|---|---|
| 5 | 1 | 4 | 5 | 5 | 5 |
| 5 | 2 | 5 | 5 | 5 | 4 |
| 4 | 1 | 4 | 4 | 4 | 5 |
| 5 | 1 | 4 | 5 | 5 | 4 |
| 5 | 1 | 5 | 5 | 5 | 5 |
| 5 | 2 | 5 | 5 | 5 | 5 |
| 5 | 1 | 5 | 5 | 4 | 5 |
| 4 | 1 | 5 | 5 | 5 | 5 |
| 5 | 1 | 5 | 5 | 5 | 5 |
| 5 | 1 | 5 | 5 | 5 | 5 |

The behavioral experiments confirm that Oxitriptan ameliorates the FXS phenotypes in FMR1 mice and could therefore be employed as a useful and efficacious treatment for FMR1 mediated autism and FXS. Furthermore, the combination of Ergoloid and Oxitriptan ameliorated all FXS phenotypes in FMR1 mice and could therefore be employed to treat autism (including FMR1 mediated autism), behavioral conditions, and FXS.

**Example 2** - **Studies relating ergoloid mesylates, oxitriptan and SSRIs to phenotypic effects in FXS and ASDs**

The experimental procedures described above for Example 1 were used in the following tests, as well as the addition of two further behaviour tests as described below. An SSRI (selective serotonin re-uptake inhibitor) was co-dosed with the ergoloid mesylates and oxitriptan to assess the effect of the ergoloid mesylates and oxitriptan treatment on a subject already receiving SSRI treatment.

*Behavioral Analysis*

Behavior testing was conducted at 2 weeks as described above for Example 1 with the addition of a resident-intruder test and a fear conditioning test.

Resident-intruder test:

Aggression is assessed in a cage in which resident subjects are habituated for several minutes. An unfamiliar animal is then introduced into the testing cage and the attack latency is measured compared to baseline values (WT and KO).

Fear conditioning test:

Mice receive several 1 second electric shocks (0.2-0.3 mA) after 1-2 minutes of habituation. During the test phase, mice are put back in the same chambers without any shock. Freezing time is measured.

*Treatment Regime*

The triple combination of ergoloid mesylates, oxitriptan and SSRI was compared to a double combination ergoloid mesylates and SSRI (as well as WT, KO controls).

The treatment of the mice with OX (Oxitriptan), ER (Ergoloid mesylates) and FL (fluvoxamine) were according to the matrix shown below in Table 21. The triple combination involved dosing of 2 mg/kg ergoloid mesylates, 40 mg/kg oxitriptan and 40 mg/kg of fluvoxamine (the SSRI).

Table 21

| Group Number | Dosing route | Dose | Dosing Regiment | No. animals |
|---|---|---|---|---|
| 1 WT | | N/A | QD | 10 |
| 2 KO | | N/A | QD | 10 |
| 3 KO ER+ FL | IP | 2 mg/kg + 40 mg/kg | QD | 10 |
| 4 KO ER+ OX+ FL | IP | 2 mg/kg + 40 mg/kg 40 mg/kg | QD | 10 |

*Results*

The results of the behavioral tests of the mice are provided in Tables 22 to 30 below.

Table 22 below shows a summary of the alleviated phenotypes with the different treatments.

Table 22

SUMMARY

| BEHAVIOUR | Erg 2 + Flu 40 | Erg 2 + Oxi 40 + Flu |
|---|---|---|
| Open field | X | ✓ |
| Nesting | ✓ | ✓ |
| Self-grooming | X | ✓ |
| Sociability | ✓ | ✓ |
| NOR | ✓ | ✓ |
| Hyponeophagia | ✓ | ✓ |
| Resident intruder | X | ✓ |
| Fear conditioning | X | ✓ |

Table 23 - Open Field (Hyperactivity) - One-way analysis of variance and multiple comparisons analysis against KO-V for Hyperactivity test (#families: 1; #comparisons: 3; alpha: 0.05).

| Dunnett's multiple comparisons test | Mean Diff. | 95.00% CI of diff. | Below threshold? | Summary | Adjusted P Value | B-? | |
|---|---|---|---|---|---|---|---|
| KO-V vs. WT-V | 2921 | 2389 to 3452 | Yes | **** | <0.0001 | A | WT-V |
| KO-V vs. KO- Erg + Flu | -749.8 | -1281 to -218.1 | Yes | ** | 0.0023 | C | KO- Erg + Flu |
| KO-V vs. KO-Erg + Oxi+Flu | 3098 | 2566 to 3629 | Yes | **** | <0.0001 | D | KO-Erg + Oxi+Flu |

| Test details | Mean 1 | Mean 2 | Mean Diff. | SE of diff. | n1 | n2 | q | DF |
|---|---|---|---|---|---|---|---|---|
| KO-V vs. WT-V | 7301 | 4381 | 2921 | 201.5 | 10 | 10 | 14.49 | 63 |
| KO-V vs. KO- Erg + Flu | 7301 | 8051 | -749.8 | 201.5 | 10 | 10 | 3.72 | 63 |
| KO-V vs. KO-Erg + Oxi+Flu | 7301 | 4204 | 3098 | 201.5 | 10 | 10 | 15.37 | 63 |

Table 24 - Nesting (Tests of daily living) - One-way analysis of variance and multiple comparisons analysis against KO-V for Test of daily living (#families: 1; #comparisons: 3; alpha: 0.05).

| Dunnett's multiple comparisons test | Mean Diff. | 95.00% CI of diff. | Below threshold? | Summary | Adjusted P Value | B-? | |
|---|---|---|---|---|---|---|---|
| KO-V vs. WT-V | -3.6 | -4.191 to -3.009 | Yes | **** | <0.0001 | A | WT-V |
| KO-V vs. KO- Erg + Flu | -3.5 | -4.091 to -2.909 | Yes | **** | <0.0001 | C | KO- Erg + Flu |
| KO-V vs. KO-Erg + Oxi+Flu | -3.4 | -3.991 to -2.809 | Yes | **** | <0.0001 | D | KO-Erg + Oxi+Flu |

| Test details | Mean 1 | Mean 2 | Mean Diff. | SE of diff. | n1 | n2 | q | DF |
|---|---|---|---|---|---|---|---|---|
| KO-V vs. WT-V | 1.2 | 4.8 | -3.6 | 0.224 | 10 | 10 | 16.07 | 63 |
| KO-V vs. KO- Erg + Flu | 1.2 | 4.7 | -3.5 | 0.224 | 10 | 10 | 15.63 | 63 |
| KO-V vs. KO-Erg + Oxi+Flu | 1.2 | 4.6 | -3.4 | 0.224 | 10 | 10 | 15.18 | 63 |

Table 25 - Partition Test (Sociability) - One-way analysis of variance and multiple comparisons analysis against KO-V for Sociability test (#families: 1; #comparisons: 3; alpha: 0.05).

| Dunnett's multiple comparisons test | Mean Diff. | 95.00% CI of diff. | Below threshold? | Summary | Adjusted P Value | D-? | |
|---|---|---|---|---|---|---|---|
| N_KO-V vs. F_WT-V | 31.5 | 26.91 to 36.09 | Yes | **** | <0.0001 | A | F_WT-V |
| N_KO-V vs. N_WT-V | -16.1 | -20.69 to -11.51 | Yes | **** | <0.0001 | B | N_WT-V |
| N_KO-V vs. F_KO-V | -0.8 | -5.392 to 3.792 | No | ns | 0.9993 | C | F_KO-V |
| N_KO-V vs. F_KO-Erg + Flu | 31.5 | 26.91 to 36.09 | Yes | **** | <0.0001 | E | F_KO- Erg + Flu |
| N_KO-V vs. N_KO-Erg + Flu | -15.8 | -20.39 to -11.21 | Yes | **** | <0.0001 | F | N_KO- Erg + Flu |
| N_KO-V vs. F_KO-Erg + Oxi+Flu | 31.5 | 26.91 to 36.09 | Yes | **** | <0.0001 | G | F_KO-Erg + Oxi+I |
| N_KO-V vs. N_KO-Erg + Oxi+Flu | -14.5 | -19.09 to -9.908 | Yes | **** | <0.0001 | H | N_KO-Erg + Oxi+ |

| Test details | Mean 1 | Mean 2 | Mean Diff. | SE of diff. | n1 | n2 | q | DF |
|---|---|---|---|---|---|---|---|---|
| N_KO-V vs. F_WT-V | 83.7 | 52.2 | 31.5 | 1.622 | 10 | 10 | 19.42 | 126 |
| N_KO-V vs. N_WT-V | 83.7 | 99.8 | -16.1 | 1.622 | 10 | 10 | 9.928 | 126 |
| N_KO-V vs. F_KO-V | 83.7 | 84.5 | -0.8 | 1.622 | 10 | 10 | 0.4933 | 126 |
| N_KO-V vs. F_KO- Erg + Flu | 83.7 | 52.2 | 31.5 | 1.622 | 10 | 10 | 19.42 | 126 |
| N_KO-V vs. N_KO- Erg + Flu | 83.7 | 99.5 | -15.8 | 1.622 | 10 | 10 | 9.743 | 126 |
| N_KO-V vs. F_KO-Erg + Oxi+Flu | 83.7 | 52.2 | 31.5 | 1.622 | 10 | 10 | 19.42 | 126 |
| N_KO-V vs. N_KO-Erg + Oxi+Flu | 83.7 | 98.2 | -14.5 | 1.622 | 10 | 10 | 8.942 | 126 |

Table 26 - Self-Grooming (Stereotypy) - One-way analysis of variance and multiple comparisons analysis against KO-V for Stereotypy test (#families: 1; #comparisons: 3; alpha: 0.05).

| Dunnett's multiple comparisons test | Mean Diff. | 95.00% CI of diff. | Below threshold? | Summary | Adjusted P Value | B-? | |
|---|---|---|---|---|---|---|---|
| KO-V vs. WT-V | 21 | 16.96 to 25.04 | Yes | **** | <0.0001 | A | WT-V |
| KO-V vs. KO- Erg + Flu | -0.5 | -4.542 to 3.542 | No | ns | 0.9995 | C | KO- Erg + Flu |
| KO-V vs. KO-Erg + Oxi+Flu | 22.5 | 18.46 to 26.54 | Yes | **** | <0.0001 | D | KO-Erg + Oxi+Flu |

| Test details | Mean 1 | Mean 2 | Mean Diff. | SE of diff. | n1 | n2 | q | DF |
|---|---|---|---|---|---|---|---|---|
| KO-V vs. WT-V | 49.7 | 28.7 | 21 | 1.532 | 10 | 10 | 13.71 | 63 |

(continued)

| Test details | Mean 1 | Mean 2 | Mean Diff. | SE of diff. | n1 | n2 | q | DF |
|---|---|---|---|---|---|---|---|---|
| KO-V vs. KO- Erg + Flu | 49.7 | 50.2 | -0.5 | 1.532 | 10 | 10 | 0.3263 | 63 |
| KO-V vs. KO-Erg + Oxi+Flu | 49.7 | 27.2 | 22.5 | 1.532 | 10 | 10 | 14.68 | 63 |

Table 27 - Novel Object Recognition (Learning and Memory) - One-way analysis of variance and multiple comparisons analysis against KO-V for NOR test (#families: 1; #comparisons: 3; alpha: 0.05).

| Dunnett's multiple comparisons test | Mean Diff. | 95.00% CI of diff. | Below threshold? | Summary | Adjusted P Value | D-? | |
|---|---|---|---|---|---|---|---|
| N_KO-V vs. F_WT-V | 1.9 | -0.03694 to 3.837 | No | ns | 0.0576 | A | F_WT-V |
| N_KO-V vs. N_WT-V | -8.3 | -10.24 to -6.363 | Yes | **** | <0.0001 | B | N_WT-V |
| N_KO-V vs. F_KO-V | -0.2 | -2.137 to 1.737 | No | ns | 0.9996 | C | F_KO-V |
| N_KO-V vs. F_KO-Erg + Flu | 0.5 | -1.437 to 2.437 | No | ns | 0.9954 | E | F_KO- Erg + Flu |
| N_KO-V vs. N_KO- Erg + Flu | -7.8 | -9.737 to -5.863 | Yes | **** | <0.0001 | F | N_KO- Erg + Flu |
| N_KO-V vs. F_KO-Erg + Oxi+Flu | -0.4 | -2.337 to 1.537 | No | ns | 0.9991 | G | F_KO-Erg + Oxi+I |
| N_KO-V vs. N_KO-Erg + Oxi+Flu | -8.2 | -10.14 to -6.263 | Yes | **** | <0.0001 | H | N_KO-Erg + Oxi+ |

| Test details | Mean 1 | Mean 2 | Mean Diff. | SE of diff. | n1 | n2 | q | DF |
|---|---|---|---|---|---|---|---|---|
| N_KO-V vs. F_WT-V | 6 | 4.1 | 1.9 | 0.6839 | 10 | 10 | 2.778 | 126 |
| N_KO-V vs. N_WT-V | 6 | 14.3 | -8.3 | 0.6839 | 10 | 10 | 12.14 | 126 |
| N_KO-V vs. F_KO-V | 6 | 6.2 | -0.2 | 0.6839 | 10 | 10 | 0.2924 | 126 |
| N_KO-V vs. F_KO- Erg + Flu | 6 | 5.5 | 0.5 | 0.6839 | 10 | 10 | 0.7311 | 126 |
| N_KO-V vs. N_KO- Erg + Flu | 6 | 13.8 | -7.8 | 0.6839 | 10 | 10 | 11.4 | 126 |
| N_KO-V vs. F_KO-Erg + Oxi+Flu | 6 | 6.4 | -0.4 | 0.6839 | 10 | 10 | 0.5848 | 126 |
| N_KO-V vs. N_KO-Erg + Oxi+Flu | 6 | 14.2 | -8.2 | 0.6839 | 10 | 10 | 11.99 | 126 |

Table 28 - Fear Conditioning - One-way analysis of variance and multiple comparisons analysis against KO-V for Fear Conditioning test (#families: 1; #comparisons: 3; alpha: 0.05).

| Dunnett's multiple comparisons test | Mean Diff. | 95.00% CI of diff. | Below threshold? | Summary | Adjusted P Value | B-? | |
|---|---|---|---|---|---|---|---|
| KO-V vs. WT-V | -25.5 | -29.02 to -21.98 | Yes | **** | <0.0001 | A | WT-V |
| KO-V vs. KO- Erg + Flu | -0.3 | -3.816 to 3.216 | No | ns | 0.9997 | C | KO- Erg + Flu |
| KO-V vs. KO-Erg + Oxi+Flu | -26.6 | -30.12 to -23.08 | Yes | **** | <0.0001 | D | KO-Erg + Oxi+Flu |

| Test details | Mean 1 | Mean 2 | Mean Diff. | SE of diff. | n1 | n2 | q | DF |
|---|---|---|---|---|---|---|---|---|
| KO-V vs. WT-V | 19.8 | 45.3 | -25.5 | 1.333 | 10 | 10 | 19.13 | 63 |

(continued)

| Test details | Mean 1 | Mean 2 | Mean Diff. | SE of diff. | n1 | n2 | q | DF |
|---|---|---|---|---|---|---|---|---|
| KO-V vs. KO- Erg + Flu | 19.8 | 20.1 | -0.3 | 1.333 | 10 | 10 | 0.2251 | 63 |
| KO-V vs. KO-Erg + Oxi+Flu | 19.8 | 46.4 | -26.6 | 1.333 | 10 | 10 | 19.96 | 63 |

Table 29 - Hyponeophagia (Anxiety) - One-way analysis of variance and multiple comparisons analysis against KO-V for Anxiety test (#families: 1; #comparisons: 3; alpha: 0.05).

| Dunnett's multiple comparisons test | Mean Diff. | 95.00% CI of diff. | Below threshold? | Summary | Adjusted P Value | B-? | |
|---|---|---|---|---|---|---|---|
| KO-V vs. WT-V | 95 | 79.00 to 111.0 | Yes | **** | <0.0001 | A | WT-V |
| KO-V vs. KO- Erg + Flu | 85.9 | 69.90 to 101.9 | Yes | **** | <0.0001 | C | KO- Erg + Flu |
| KO-V vs. KO-Erg + Oxi+Flu | 64.8 | 48.80 to 80.80 | Yes | **** | <0.0001 | D | KO-Erg + Oxi+Flu |

| Test details | Mean 1 | Mean 2 | Mean Diff. | SE of diff. | n1 | n2 | q | DF |
|---|---|---|---|---|---|---|---|---|
| KO-V vs. WT-V | 232.8 | 137.8 | 95 | 6.065 | 10 | 10 | 15.66 | 63 |
| KO-V vs. KO- Erg + Flu | 232.8 | 146.9 | 85.9 | 6.065 | 10 | 10 | 14.16 | 63 |
| KO-V vs. KO-Erg + Oxi+Flu | 232.8 | 168 | 64.8 | 6.065 | 10 | 10 | 10.68 | 63 |

Table 30 - Resident Intruder (Aggression) - One-way analysis of variance and multiple comparisons analysis against KO-V for Aggression test (#families: 1; #comparisons: 3; alpha: 0.05).

| Dunnett's multiple comparisons test | Mean Diff. | 95.00% CI of diff. | Below threshold? | Summary | Adjusted P Value | B-? | |
|---|---|---|---|---|---|---|---|
| KO-V vs. WT-V | -48.8 | -55.74 to -41.86 | Yes | **** | <0.0001 | A | WT-V |
| KO-V vs. KO- Erg + Flu | -54.2 | -61.14 to -47.26 | Yes | **** | <0.0001 | C | KO- Erg + Flu |
| KO-V vs. KO-Erg + Oxi+Flu | -53.6 | -60.54 to -46.66 | Yes | **** | <0.0001 | D | KO-Erg + Oxi+Flu |

| Test details | Mean 1 | Mean 2 | Mean Diff. | SE of diff. | n1 | n2 | q | DF |
|---|---|---|---|---|---|---|---|---|
| KO-V vs. WT-V | 140.2 | 189 | -48.8 | 2.629 | 10 | 10 | 18.56 | 63 |
| KO-V vs. KO- Erg + Flu | 140.2 | 194.4 | -54.2 | 2.629 | 10 | 10 | 20.61 | 63 |
| KO-V vs. KO-Erg + Oxi+Flu | 140.2 | 193.8 | -53.6 | 2.629 | 10 | 10 | 20.39 | 63 |

These behavioral experiments confirm that a tryptophan such as oxitriptan, in combination with ergoloid mesylates, ameliorates the FXS phenotypes in FMR1 mice when simultaneously administered with the SSRI fluvoxamine, and could therefore be employed as a useful and efficacious treatment for FXS and FMR1 mediated autism in patients already receiving SSRIs such as fluvoxamine.

**Example 3**

Example Formulations and Treatments

A number of example formulations are provided below along with suggested dosage regimes. It will be understood that these are for illustrative purposes and these would be optimized during further experimentation, which

may include clinical trials. For simplicity, the formulations do not stipulate any non-active components (such as pharmaceutically acceptable carriers or excipients etc.)

***Formulation 3A - Oxitriptan - Oral Tablet for the Treatment FMR1 Mediated Autism***

| Active Ingredient | Form | mg | Dose |
|---|---|---|---|
| Oxitriptan | Oral Tablet | 200 | Once daily |

***Formulation 3B - Oxitriptan - Oral Tablet for the Treatment FMR1 Mediated Autism***

| Active Ingredient | Form | mg | Dose |
|---|---|---|---|
| Oxitriptan | Oral Tablet | 100 | Three times daily |

***Formulation 3C - Oxitriptan - Oral Tablet for the Treatment Fragile X Syndrome (FXS)***

| Active Ingredient | Form | mg | Dose |
|---|---|---|---|
| Oxitriptan | Oral Tablet | 200 | Once daily |

***Formulation 3D - Oxitriptan + Ergoloid Mesylates - Oral Tablets for the Treatment of Autism***

| Active Ingredient | Form | mg | Dose |
|---|---|---|---|
| Oxitriptan | Oral Tablet | 200 | Once daily |
| Ergoloid Mesylates | Oral Tablet | 2 | Once daily |

***Formulation 3E - Oxitriptan + Ergoloid Mesylates - Oral Tablets for the Treatment of FMR1 mediated Autism***

| Active Ingredient | Form | mg | Dose |
|---|---|---|---|
| Oxitriptan | Oral Tablet | 200 | Once daily |
| Ergoloid Mesylates | Oral Tablet | 2 | Once daily |

***Formulation 3F - Oxitriptan + Ergoloid Mesylates - Oral Tablets for the Treatment of Fragile X Syndrome (FXS)***

| Active Ingredient | Form | mg | Dose |
|---|---|---|---|
| Oxitriptan | Oral Tablet | 200 | Once daily |
| Ergoloid Mesylates | Oral Tablet | 2 | Once daily |

***Formulation 3G - Oxitriptan + Ergoloid Mesylates - Oral Tablets for the Treatment of Autism, FMR1 mediated Autism and/or Fragile X Syndrome (FXS)***

| Active Ingredient | Form | mg | Dose |
|---|---|---|---|
| Oxitriptan | Oral Tablet | 100 | Three times daily |
| Ergoloid Mesylates | Oral Tablet | 1 | Three times daily |

The skilled addressee will of course understand that therapeutically effective doses will of course depend on the activity and format of the chosen pharmaceutically active ingredient.

The forgoing embodiments are not intended to limit the scope of the protection afforded by the claims, but rather to describe examples of how the invention may be put into practice.

References

Thompson TL 2nd, Filley CM, Mitchell WD, Culig KM, LoVerde M, Byyny RL: Lack of efficacy of hydergine in patients with Alzheimer's disease. N Engl J Med. 1990 Aug 16;323(7):445-8. doi: 10.1056/NEJM199008163230704. [PubMed:2082953]

PERCHESON PB, CARROLL JJ: The use of hydergine in obstetrics. Can Med Assoc J. 1954 Dec;71(6):588-94. [PubMed:13209453]

Pillay V.V. (2013). Modern medical toxicology (4th ed.). Jaypee Brothers. Barceloux D. (2008). Medical toxicology of natural substances. Wiley. [ISBN:978-0-470-33447-4]

Seyffart G. (1992). Drug dosage in renal insufficiency (2nd ed.). Springer Science+Business Media Dordrecht.

HAFKENSCHIEL JH, CRUMPTON CW, MOYER JH, JEFFERS WA, FISHEL HANLEY B, CONLIN HARNED S: The effects of dihydroergocornine on the cerebral circulation of patients with essential hypertension. J Clin Invest. 1950 Apr;29(4):408-11. doi: 10.1172/JC1102273. [PubMed:15415441]

FREIS ED, STANTON JR, et al.: The hemodynamic effects of hypotensive drugs in man; dihydroergocornine. J Clin Invest. 1949 Nov;28(6 Pt 2):1387-1402. doi: 10.1172/JCI102204. [PubMed:15395942]

Bercel NA: TREATMENT OF MIGRAINE-Results with Dihydroergocornine Methanesulfonate (DHO-180) and Other Ergot Derivatives. Calif Med. 1950 Apr;72(4):234-8. [PubMed:18731688]

Jacobsen JPR, Krystal AD, Krishnan KRR, Caron MG. Adjunctive 5-Hydroxytryptophan Slow-Release for Treatment-Resistant Depression: Clinical and Preclinical Rationale. Trends Pharmacol Sci. 2016;37(11):933-944. doi:10.1016/j.tips.2016.09.001

Hawkins M. A 10-year history of using 5-Hydroxytryptophan for severe insomnia in a 15-year-old with autism, seizures, and sleep apnea: cause for concern? Abstracts / Sleep Medicine 64 (2019) S1-S359.

Titus, Feliu, et al. "5-hydroxytryptophan versus methysergide in the prophylaxis of migraine." European neurology 25.5 (1986): 327-329.

Drummond, P. D. "Tryptophan depletion increases nausea, headache and photophobia in migraine sufferers." Cephalalgia 26.10 (2006): 1225-1233.

Duquesnoy C, Mamet JP, Sumner D, Fuseau E. Comparative clinical pharmacokinetics of single doses of sumatriptan following subcutaneous, oral, rectal and intranasal administration. Eur J Pharm Sci. 1998;6(2):99-104. doi:10.1016/s0928-0987(97)00073-0 Boccuto L, Chen CF, Pittman AR, et al. Decreased tryptophan metabolism

in patients with autism spectrum disorders. Mol Autism. 2013;4(1):16. Published 2013 Jun 3. doi:10.1186/2040-2392-4-16

Costa L, Spatuzza M, D'Antoni S, et al. Activation of 5-HT7 serotonin receptors reverses metabotropic glutamate receptor-mediated synaptic plasticity in wild-type and Fmr1 knockout mice, a model of Fragile X syndrome. Biol Psychiatry. 2012;72(11):924-933. doi:10.1016/j.biopsych.2012.06.008

Costa L et al, Novel agonists for serotonin 5-HT7 receptors reverse metabotropic glutamate receptor-mediated long-term depression in the hippocampus of wild-type and Fmr1 KO mice, a model of Fragile X Syndrome. Front Behav Neurosci. 2015; 9: 65., doi: 10.3389/fnbeh.2015.00065

Hanson AC, Hagerman RJ. Serotonin dysregulation in Fragile X Syndrome: implications for treatment. Intractable Rare Dis Res. 2014;3(4):110-117. doi:10.5582/irdr.2014.01027

Sverd J, Kupietz SS, Winsberg BG, Hurwic MJ, Becker L. Effects of L-5-hydroxytryptophan in autistic children. J Autism Child Schizophr. 1978;8(2):171-180. doi:10.1007/BF01537866

Ritvo, E.R., Yuwiler, A., Geller, E. et al. Effects of L-dopa in autism. J Autism Dev Disord 1, 190-205 (1971). https://doi.org/10.1007/BF01537957

Novotny, S., Hollander, E., Allen, A., Mosovich, S., Aronowitz, B., Cartwright, C., . . . Dolgoff-Kaspar, R. (2000). Increased growth hormone response to sumatriptan challenge in adult autistic disorders. Psychiatry Research, 94(2), 173-177. doi:10.1016/s0165-1781(00)00134-7

Hollander, E., Novotny, S., Allen, A. et al. The Relationship between Repetitive Behaviors and Growth Hormone Response to Sumatriptan Challenge in Adult Autistic Disorder. Neuropsychopharmacol 22, 163-167 (2000).https://doi.org/10.1016/S0893-133X(99)00121-9

Gurney ME et al.,Multiple Behavior Phenotypes of the Fragile-X Syndrome Mouse Model Respond to Chronic Inhibition of Phosphodiesterase-4D (PDE4D). Sci Rep 2017 Nov 7;7(1):14653. doi: 10.1038/s41598-017-15028-x.

Kalueff, Allan V., et al. "Neurobiology of rodent self-grooming and its value for translational neuroscience." Nature Reviews Neuroscience 17.1 (2016): 45.

Gaskill, Brianna N., et al. "Nest building as an indicator of health and welfare in laboratory mice." JoVE (Journal of Visualized Experiments) 82 (2013): e51012.

Deacon, Rob MJ. "Hyponeophagia: a measure of anxiety in the mouse." JoVE (Journal of Visualized Experiments) 51 (2011): e2613.

Antunes, M., and Grazyna Biala. "The novel object recognition memory: neurobiology, test procedure, and its modifications." Cognitive processing 13.2 (2012): 93-110.

Kaidanovich-Beilin, Oksana, et al. "Assessment of social interaction behaviors." JoVE (Journal of Visualized Experiments) 48 (2011): e2473.

Gould, Todd D., David T. Dao, and Colleen E. Kovacsics. "The open field test." Mood and anxiety related phenotypes in mice. Humana Press, Totowa, NJ, 2009. 1-20.

Razak, Khaleel A., Kelli C. Dominick, and Craig A. Erickson. "Developmental studies in fragile X syndrome." Journal of Neurodevelopmental Disorders 12 (2020): 1-15.

Hunter, Jessica, et al. "Epidemiology of fragile X syndrome: A systematic review and meta-analysis." American Journal of Medical Genetics Part A 164.7 (2014): 1648-1658.

Hagerman, Randi J. "Psychopharmacological interventions in fragile X syndrome, fetal alcohol syndrome, Prader-Willi syndrome, Angelman syndrome, Smith-Magenis syndrome, and velocardiofacial syndrome." Mental Retardation and Developmental Disabilities Research Reviews 5.4 (1999): 305-313.

Suhl, J.A. and Warren, S.T., 2015. Single-nucleotide mutations in FMR1 reveal novel functions and regulatory mechanisms of the fragile X syndrome protein FMRP. Journal of experimental neuroscience, 9, pp.JEN-S25524.

Kalra, V., Seth, R. and Sapra, S., 2005. Autism - experiences in a tertiary care hospital. The Indian Journal of Pediatrics, 72(3), pp.227-230.

Dahlhaus R, Of Men and Mice: Modeling the Fragile X Syndrome, frontiers in Molecular NeuroScience, 2018, doi: 10.3389/fnmol.2018.00041

Bakker et al, The Dutch-Belgian Fragile X Consortium (1994) Fmr1 knockout mice: A model to study fragile X mental retardation Cell 78(1):23-33.

Yan, QJ, Asafo-Adjei, PK, Arnold, HM, Brown, RE, and Bauchwitz, RP (2004). A phenotypic and molecu-lar characterization of the fmr1-tm1Cgr Fragile X mouse. Genes, Brain and Behavior 3(6): 337-359.

Mientjes, EJ et al. (2006). The generation of a conditional Fmr1 knockout mouse model to study Fmrp function in vivo. Neurobiology of Disease 21(3): 549-555.

Liu XS, Wu H, Krzisch M, Wu X, Graef J, Muffat J, Hnisz D, Li CH, Yuan B, Xu C, Li Y. Rescue of fragile X syndrome neurons by DNA methylation editing of the FMR1 gene. Cell. 2018 Feb 22;172(5):979-92.

Mines MA, Yuskaitis CJ, King MK, Beurel E, Jope RS. GSK3 influences social preference and anxiety-related behaviors during social interaction in a mouse model of fragile X syndrome and autism. PLoS One. 2010;5(3):e9706. Published 2010 Mar 16. doi:10.1371/journal.pone.0009706

Sørensen EM, Bertelsen F, Weikop P, et al. Hyperactivity and lack of social discrimination in the adolescent Fmr1 knockout mouse. Behav Pharmacol. 2015;26(8 Spec No):733-740. doi:10.1097/FBP.0000000000000152

Bernardet M, Crusio WE. Fmr1 KO mice as a possible model of autistic features. ScientificWorldJournal. 2006;6:1164-1176. Published 2006 Sep 20. doi:10.1100/tsw.2006.220

Arsenault J, Gholizadeh S, Niibori Y, et al. FMRP Expression Levels in Mouse Central Nervous System Neurons Determine Behavioral Phenotype. Hum Gene Ther. 2016;27(12):982-996. doi:10.1089/hum.2016.090

Dölen G, Bear MF. Fragile x syndrome and autism: from disease model to therapeutic targets. J Neurodev Disord. 2009;1(2):133-140. doi:10.1007/s11689-009-9015-x

Hodges SL, Nolan SO, Taube JH, Lugo JN. Adult Fmr1 knockout mice present with deficiencies in hippocampal interleukin-6 and tumor necrosis factor-α expression. Neuroreport. 2017;28(18):1246-1249. doi:10.1097/WNR.0000000000000905

Domanski, A.P.F., Booker, S.A., Wyllie, D.J.A. et al. Cellular and synaptic phenotypes lead to disrupted information processing in Fmr1-KO mouse layer 4 barrel cortex. Nat Commun 10, 4814 (2019). https://doi.org/10.1038/s41467-019-12736-y

Arbab, T., Pennartz, C.M.A. & Battaglia, F.P. Impaired hippocampal representation of place in the Fmr1-knockout mouse model of fragile X syndrome. Sci Rep 8, 8889 (2018). https://doi.org/10.1038/s41598-018-26853-z

Filonova, Irina, "Ube3a Role in Synaptic Plasticity and Neurodevelopmental Disorders.The Lessons from Angelman Syndrome." (2014). Graduate Theses and Dissertations. https://scholarcommons.usf.edu/etd/5015

Couvert, P., Bienvenu, T., Aquaviva, C., Poirier, K., Moraine, C., Gendrot, C., Verloes, A., Andrès, C., Le Fevre, A.C., Souville, I. and Steffann, J., 2001. MECP2 is highly mutated in X-linked mental retardation. Human Molecular Genetics, 10(9), pp.941-946.

Khatri, N. and Man, H.Y., 2019. The autism and Angelman syndrome protein Ube3A/E6AP: The gene, E3 ligase ubiquitination targets and neurobiological functions. Frontiers in Molecular Neuroscience, 12, p.109.

## Claims

1. A composition for use in the treatment, management or amelioration of FMR1 mediated autism, wherein the composition comprises one or more tryptophans selected from 5-Hydroxytryptophan (5-HTP) (Oxitriptan), Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

2. A composition for use in the treatment, management or amelioration of Fragile X Syndrome (FXS), wherein the composition comprises one or more tryptophans selected from 5-Hydroxytryptophan (5-HTP) (Oxitriptan), Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

3. The composition for use as claimed in claim 1 or claim 2, wherein the one or more tryptophans comprises 5-Hydroxytryptophan (5-HTP) (Oxitriptan).

4. The composition for use as claimed in claim 1 or claim 2, wherein the one or more tryptophans are selected from: Tryptophan (L-Tryptophan); D-Tryptophan; 2-Hydroxy-Tryptophan; Imino-Tryptophan; Indoximod; 1-hydroperoxy-L-tryptophan; Tryptophanamide; 5-fluorotryptophan; 7-Aza-L-tryptophan; and Oglufanide or mixtures thereof.

5. The composition for use as claimed in any preceding claim, wherein the composition comprises one or more ergot alkaloids.

6. The composition for use as claimed in claim 5, wherein the one or more ergot alkaloids comprises ergoloid mesylates.

7. The composition for use as claimed in claim 6, wherein the one or more ergot alkaloids comprises a substantially equiproportional preparation of dihydroergocornine, dihydroergocristine, and dihydroergocryptine.

8. The composition for use as claimed in claim 5, wherein the one or more tryptophans and the one or more ergot alkaloids are in a mixture.

9. The composition for use as claimed in claim 5, wherein the one or more tryptophans are for administration separately, together or sequentially with the one or more ergot alkaloids.

10. The composition for use as claimed in any one of claims 5 to 7, comprising oxitriptan and ergoloid mesylates, wherein the composition is administered in a daily dose in the range of about 20 mg to about 400 mg of oxitriptan and in the range of about 1 mg to about 3mg of ergoloid mesylates.

11. The composition for use as claimed in claim 10, wherein the composition is administered in a dose of about 100 mg TID of oxitriptan and a dose of about 1 mg TID of ergoloid mesylates.

12. The composition for use of claim 1, the composition comprising an SSRI.

## Patentansprüche

1. Zusammensetzung zur Verwendung bei der Behandlung, dem Management oder der Besserung von FMR1-vermitteltem Autismus, wobei die Zusammensetzung ein oder mehrere Tryptophane umfasst, die aus 5-Hydroxytryptophan (5-HTP) (Oxitriptan), Tryptophan (L-Tryptophan); D-Tryptophan; 2-Hydroxytryptophan; Iminotryptophan; Indoximod; 1-Hydroperoxy-L-tryptophan; Tryptophanamid; 5-Fluortryptophan; 7-Aza-L-tryptophan und Oglufanid oder Mischungen davon ausgewählt sind.

2. Zusammensetzung zur Verwendung bei der Behandlung, dem Management oder der Besserung von Fragiles-X-Syndrom (FXS), wobei die Zusammensetzung ein oder mehrere Tryptophane umfasst, die aus 5-Hydroxytryptophan (5-HTP) (Oxitriptan), Tryptophan (L-Tryptophan); D-Tryptophan; 2-HydroxytTryptophan; Iminotryptophan; Indoximod; 1-Hydroperoxy-L-tryptophan; Tryptophanamid; 5-Fluortryptophan; 7-Aza-L-tryptophan und Oglufanid oder Mischungen davon ausgewählt sind.

3. Zusammensetzung zur Verwendung nach Anspruch 1 oder Anspruch 2, wobei das eine oder die mehreren

Tryptophane 5-Hydroxytryptophan (5-HTP) (Oxitriptan) umfassen.

4. Zusammensetzung zur Verwendung nach Anspruch 1 oder Anspruch 2, wobei das eine oder die mehreren Tryptophane ausgewählt sind aus: Tryptophan (L-Tryptophan); D-Tryptophan; 2-Hydroxytryptophan; Iminotryptophan; Indoximod; 1-Hydroperoxy-L-tryptophan; Tryptophanamid; 5-Fluortryptophan; 7-Aza-L-tryptophan und Oglufanid oder Mischungen davon.

5. Zusammensetzung zur Verwendung nach einem der vorhergehenden Ansprüche, wobei die Zusammensetzung ein oder mehrere Ergotalkaloide umfasst.

6. Zusammensetzung zur Verwendung nach Anspruch 5, wobei das eine oder die mehreren Ergotalkaloide Ergoloidmesylate umfassen.

7. Zusammensetzung zur Verwendung nach Anspruch 6, wobei das eine oder die mehreren Ergotalkaloide eine weitgehend äquiproportionale Zubereitung von Dihydroergocornin, Dihydroergocristin und Dihydroergocryptin umfassen.

8. Zusammensetzung zur Verwendung nach Anspruch 5, wobei das eine oder die mehreren Tryptophane und das eine oder die mehreren Ergotalkaloide in einer Mischung vorliegen.

9. Zusammensetzung zur Verwendung nach Anspruch 5, wobei das eine oder die mehreren Tryptophane für die separate, gemeinsame oder aufeinanderfolgende Verabreichung mit dem einen oder den mehreren Ergotalkaloiden vorgesehen ist.

10. Zusammensetzung zur Verwendung nach einem der Ansprüche 5 bis 7, umfassend Oxitriptan und Ergoloidmesylate, wobei die Zusammensetzung in einer Tagesdosis im Bereich von etwa 20 mg bis etwa 400 mg Oxitriptan und im Bereich von etwa 1 mg bis etwa 3 mg Ergoloidmesylaten verabreicht wird.

11. Zusammensetzung zur Verwendung nach Anspruch 10, wobei die Zusammensetzung in einer Dosis von etwa 100 mg TID Oxitriptan und einer Dosis von etwa 1 mg TID Ergoloidmesylaten verabreicht wird.

12. Zusammensetzung zur Verwendung nach Anspruch 1, wobei die Zusammensetzung einen SSRI umfasst.

## Revendications

1. Composition pour utilisation dans le traitement, la gestion ou l'amélioration de l'autisme médié par FMR1, dans laquelle la composition comprend un ou plusieurs tryptophanes choisis parmi 5-Hydroxytryptophane (5-HTP) (Oxitriptan), Tryptophane (L-Tryptophane) ; D-Tryptophane ; 2-Hydroxy-Tryptophane ; Imino-Tryptophane ; Indoximod ; 1-hydroperoxy-L-tryptophane ; Tryptophanamide ; 5-fluorotryptophane ; 7-Aza-L-tryptophane ; et Oglufanide ou des mélanges correspondants.

2. Composition pour utilisation dans le traitement, la gestion ou l'amélioration du syndrome de l'X fragile (FXS), dans laquelle la composition comprend un ou plusieurs tryptophanes choisis parmi 5-Hydroxytryptophane (5-HTP) (Oxitriptan), Tryptophane (L-Tryptophane) ; D-Tryptophane ; 2-Hydroxy-Tryptophane ; Imino-Tryptophane ; Indoximod ; 1-hydroperoxy-L-tryptophane ; Tryptophanamide ; 5-fluorotryptophane ; 7-Aza-L-tryptophane ; et Oglufanide ou des mélanges correspondants.

3. Composition pour utilisation selon la revendication 1 ou la revendication 2, dans laquelle le ou les tryptophanes comprennent 5-Hydroxytryptophane (5-HTP) (Oxitriptan).

4. Composition pour utilisation selon la revendication 1 ou la revendication 2, dans laquelle le ou les tryptophanes sont choisis parmi : Tryptophane (L-Tryptophane) ; D-Tryptophane ; 2-Hydroxy-Tryptophane ; Imino-Tryptophane ; Indoximod ; 1-hydroperoxy-L-tryptophane ; Tryptophanamide ; 5-fluorotryptophane ; 7-Aza-L-tryptophane ; et Oglufanide ou des mélanges correspondants.

5. Composition pour utilisation selon l'une quelconque des revendications précédentes, dans laquelle la composition comprend un ou plusieurs alcaloïdes de l'ergot.

**6.** Composition pour utilisation selon la revendication 5, dans laquelle le ou les alcaloïdes de l'ergot comprennent des mésylates ergoloïdes.

**7.** Composition pour utilisation selon la revendication 6, dans laquelle le ou les alcaloïdes de l'ergot comprennent une préparation sensiblement équiproportionnelle de dihydroergocornine, dihydroergocristine et dihydroergocryptine.

**8.** Composition pour utilisation selon la revendication 5, dans laquelle le ou les tryptophanes et le ou les alcaloïdes de l'ergot sont dans un mélange.

**9.** Composition pour utilisation selon la revendication 5, dans laquelle le ou les tryptophanes sont destinés à être administrés séparément, conjointement ou séquentiellement avec le ou les alcaloïdes de l'ergot.

**10.** Composition pour utilisation selon l'une quelconque des revendications 5 à 7, comprenant de l'oxitriptan et des mésylates ergoloïdes, dans laquelle la composition est administrée en une dose quotidienne dans la plage d'environ 20 mg à environ 400 mg d'oxitriptan et dans la plage d'environ 1 mg à environ 3 mg de mésylates ergoloïdes.

**11.** Composition pour utilisation selon la revendication 10, dans laquelle la composition est administrée en une dose d'environ 100 mg TID d'oxitriptan et en une dose d'environ 1 mg TID de mésylates ergoloïdes.

**12.** Composition pour utilisation selon la revendication 1, la composition comprenant un SSRI.

Figure 1

10000
8000
6000
4000
2000
0
Distance travelled (cm)
WT-V
KO-V
KO-Sumatriptan (20 mg/kg)
KO-Oxitriptan (80 mg/kg)
KO-Ergoloid (4 mg/kg)
KO - Ergoloid (2 mg/kg) + Oxitriptan (40 mg/kg)

Figure 2

60
40
20
0
Self-grooming
WT-V
KO-V
KO-Sumatriptan (20 mg/kg)
KO-Oxitriptan (40 mg/kg)
KO-Ergoloid (4 mg/kg)
KO - Ergoloid (2 mg/kg) + Oxitriptan (40 mg/kg)

Figure 3

150
100
50
0
Time spent with the novel mouse (S)
F_WT-V
N_WT-V
F_KO-V
N_KO-V
F_KO-Sumatriptan (20 mg/kg)
N_KO-Sumatriptan (20 mg/kg)
F_KO-Oxitriptan (40 mg/kg)
N_KO-Oxitriptan (40 mg/kg)
F_KO-Ergoloid (4 mg/kg)
N_KO-Ergoloid (4 mg/kg)
F_KO - Ergoloid (2 mg/kg) + Oxitriptan (40 mg/kg)
N_KO - Ergoloid (2 mg/kg) + Oxitriptan (40 mg/kg)

Figure 4

Time spent with the novel object (S)
20
15
10
5
0
F_WT-V
N_WT-V
F_KO-V
N_KO-V
F_KO-Sumatriptan (20 mg/kg)
N_KO-Sumatriptan (20 mg/kg)
F_KO-Oxitriptan (40 mg/kg)
N_KO-Oxitriptan (40 mg/kg)
F_KO-Ergoloid (4 mg/kg)
N_KO-Ergoloid (4 mg/kg)
F_KO - Ergoloid (2 mg/kg) + Oxitriptan (40 mg/kg)
N_KO - Ergoloid (2 mg/kg) + Oxitriptan (40 mg/kg)

Figure 5

300
200
100
0
Latency (s)
WT-V
KO-V
KO-Sumatriptan (20 mg/kg)
KO-Oxitriptan (40 mg/kg)
KO-Ergoloid (4 mg/kg)
KO - Ergoloid (2 mg/kg) + Oxitriptan (40 mg/kg)

Figure 6

6
4
2
0
Nest Building quality
(grade 1 to 5)
WT-V
KO-V
KO-Sumatriptan (20 mg/kg)
KO-Oxitriptan (40 mg/kg)
KO-Ergoloid (4 mg/kg)
KO - Ergoloid (2 mg/kg) + Oxitriptan (40 mg/kg)

Figure 7

10000
8000
6000
4000
2000
0
Distance travelled (cm)
WT-V
KO-V
KO-Erg + Flu
KO-Erg + Oxi + Flu

Figure 8

6
4
2
0
Nest Building quality (grade 1 to 5)
WT-V
KO-V
KO-Erg + Flu
KO-Erg + Oxi + Flu

**Figure 9**

Time spent with the novel mouse (S)
150
100
50
0
F_WT-V
N_WT-V
F_KO-V
F_WT-V
F_KO-Erg + Flu
N_KO-Erg + Flu
F_KO-Erg + Oxi + Flu
N_KO-Erg + Oxi + Flu


**Figure 10**

Self-grooming
60
40
20
0
WT-V
KO-V
KO-Erg + Flu
KO-Erg + Oxi + Flu

**Figure 11**

Latency (s)
300
200
100
0
WT-V
KO-V
KO-Erg + Flu
KO-Erg + Oxi + Flu


**Figure 12**

Time spent with the novel object (S)
20
15
10
5
0
F_WT-V
N_WT-V
F_KO-V
F_WT-V
F_KO-Erg + Flu
N_KO-Erg + Flu
F_KO-Erg + Oxi + Flu
N_KO-Erg + Oxi + Flu

**Figure 13**

60
40
20
0
Latency (s)
WT-V
KO-V
KO-Erg + Flu
KO-Erg + Oxi + Flu

**Figure 14**

250
200
150
100
50
0
Attack Latency Time (s)
WT-V
KO-V
KO-Erg + Flu
KO-Erg + Oxi + Flu

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5885976 A
- US 9468627 B2


### Non-patent literature cited in the description


- **THOMPSON TL 2ND** ; **FILLEY CM** ; **MITCHELL WD** ; **CULIG KM** ; **LOVERDE M** ; **BYYNY RL**. Lack of efficacy of hydergine in patients with Alzheimer's disease. *N Engl J Med.*, 16 August 1990, vol. 323 (7), 445-8
- **PERCHESON PB** ; **CARROLL JJ**. The use of hydergine in obstetrics. *Can Med Assoc J.*, December 1954, vol. 71 (6), 588-94
- **PILLAY V.V.** Modern medical toxicology. Jaypee Brothers, 2013
- **BARCELOUX D.** Medical toxicology of natural substances. Wiley, 2008
- **SEYFFART G.** Drug dosage in renal insufficiency. Springer Science+Business Media, 1992
- **HAFKENSCHIEL JH** ; **CRUMPTON CW** ; **MOYER JH** ; **JEFFERS WA** ; **FISHEL HANLEY B** ; **CONLIN HARNED S**. The effects of dihydroergocornine on the cerebral circulation of patients with essential hypertension. *J Clin Invest.*, April 1950, vol. 29 (4), 408-11
- **FREIS ED** ; **STANTON JR et al.** The hemodynamic effects of hypotensive drugs in man; dihydroergocornine. *J Clin Invest.*, November 1949, vol. 28 (6), 1387-1402
- **BERCEL NA**. TREATMENT OF MIGRAINE-Results with Dihydroergocornine Methanesulfonate (DHO-180) and Other Ergot Derivatives. *Calif Med.*, April 1950, vol. 72 (4), 234-8
- **JACOBSEN JPR** ; **KRYSTAL AD** ; **KRISHNAN KRR** ; **CARON MG**. Adjunctive 5-Hydroxytryptophan Slow-Release for Treatment-Resistant Depression: Clinical and Preclinical Rationale. *Trends Pharmacol Sci.*, 2016, vol. 37 (11), 933-944
- **HAWKINS M.** A 10-year history of using 5-Hydroxytryptophan for severe insomnia in a 15-year-old with autism, seizures, and sleep apnea: cause for concern?. *Abstracts / Sleep Medicine*, 2019, vol. 64, S1-S359
- **TITUS, FELIU et al.** 5-hydroxytryptophan versus methysergide in the prophylaxis of migraine.. *European neurology*, 1986, vol. 25 (5), 327-329
- **DRUMMOND, P. D.** Tryptophan depletion increases nausea, headache and photophobia in migraine sufferers.. *Cephalalgia*, 2006, vol. 26 (10), 1225-1233
- **DUQUESNOY C** ; **MAMET JP** ; **SUMNER D** ; **FUSEAU E.** Comparative clinical pharmacokinetics of single doses of sumatriptan following subcutaneous, oral, rectal and intranasal administration. *Eur J Pharm Sci.*, 1998, vol. 6 (2), 99-104
- **BOCCUTO L** ; **CHEN CF** ; **PITTMAN AR et al.** Decreased tryptophan metabolism in patients with autism spectrum disorders. *Mol Autism*, 03 June 2013, vol. 4 (1), 16
- **COSTA L** ; **SPATUZZA M** ; **D'ANTONI S et al.** Activation of 5-HT7 serotonin receptors reverses metabotropic glutamate receptor-mediated synaptic plasticity in wild-type and Fmr1 knockout mice, a model of Fragile X syndrome. *Biol Psychiatry*, 2012, vol. 72 (11), 924-933
- **COSTA L et al.** Novel agonists for serotonin 5-HT7 receptors reverse metabotropic glutamate receptor-mediated long-term depression in the hippocampus of wild-type and Fmr1 KO mice, a model of Fragile X Syndrome. *Front Behav Neurosci.*, 2015, vol. 9, 65
- **HANSON AC** ; **HAGERMAN RJ**. Serotonin dysregulation in Fragile X Syndrome: implications for treatment. *Intractable Rare Dis Res.*, 2014, vol. 3 (4), 110-117
- **SVERD J** ; **KUPIETZ SS** ; **WINSBERG BG** ; **HURWIC MJ** ; **BECKER L**. Effects of L-5-hydroxytryptophan in autistic children. *J Autism Child Schizophr.*, 1978, vol. 8 (2), 171-180
- **RITVO, E.R.** ; **YUWILER, A.** ; **GELLER, E. et al.** Effects of L-dopa in autism. *J Autism Dev Disord*, 1971, vol. 1, 190-205, https://doi.org/10.1007/BF01537957
- **NOVOTNY, S.** ; **HOLLANDER, E.** ; **ALLEN, A.** ; **MOSOVICH, S.** ; **ARONOWITZ, B.** ; **CARTWRIGHT, C.** ; **DOLGOFF-KASPAR, R.** Increased growth hormone response to sumatriptan challenge in adult autistic disorders. *Psychiatry Research*, 2000, vol. 94 (2), 173-177

- **HOLLANDER, E.** ; **NOVOTNY, S.** ; **ALLEN, A. et al.** The Relationship between Repetitive Behaviors and Growth Hormone Response to Sumatriptan Challenge in Adult Autistic Disorder. *Neuropsychopharmacol*, 2000, vol. 22, 163-167, https://doi.org/10.1016/S0893-133X(99)00121-9
- **GURNEY ME et al.** Multiple Behavior Phenotypes of the Fragile-X Syndrome Mouse Model Respond to Chronic Inhibition of Phosphodiesterase-4D (PDE4D). *Sci Rep*, 07 November 2017, vol. 7 (1), 14653
- **KALUEFF, ALLAN V. et al.** Neurobiology of rodent self-grooming and its value for translational neuroscience.. *Nature Reviews Neuroscience*, 2016, vol. 17 (1), 45
- **GASKILL, BRIANNA N. et al.** Nest building as an indicator of health and welfare in laboratory mice.. *JoVE (Journal of Visualized Experiments)*, 2013, vol. 82, e51012
- **DEACON, ROB MJ.** Hyponeophagia: a measure of anxiety in the mouse.. *JoVE (Journal of Visualized Experiments)*, 2011, vol. 51, e2613
- **ANTUNES, M.** ; **GRAZYNA BIALA**. The novel object recognition memory: neurobiology, test procedure, and its modifications.. *Cognitive processing*, 2012, vol. 13 (2), 93-110
- **KAIDANOVICH-BEILIN, OKSANA et al.** Assessment of social interaction behaviors.. *JoVE (Journal of Visualized Experiments)*, 2011, vol. 48, e2473
- The open field test.. **GOULD, TODD D.** ; **DAVID T. DAO** ; **COLLEEN E. KOVACSICS**. Mood and anxiety related phenotypes in mice. Humana Press, 2009, 1-20
- **RAZAK, KHALEEL A.** ; **KELLI C. DOMINICK** ; **CRAIG A. ERICKSON**. Developmental studies in fragile X syndrome.. *Journal of Neurodevelopmental Disorders*, 2020, vol. 12, 1-15
- **HUNTER, JESSICA et al.** Epidemiology of fragile X syndrome: A systematic review and meta-analysis. *American Journal of Medical Genetics Part A*, 2014, vol. 164 (7), 1648-1658
- **HAGERMAN, RANDI J.** Psychopharmacological interventions in fragile X syndrome, fetal alcohol syndrome, Prader-Willi syndrome, Angelman syndrome, Smith-Magenis syndrome, and velocardiofacial syndrome.. *Mental Retardation and Developmental Disabilities Research Reviews*, 1999, vol. 5 (4), 305-313
- **SUHL, J.A.** ; **WARREN, S.T.** Single-nucleotide mutations in FMR1 reveal novel functions and regulatory mechanisms of the fragile X syndrome protein FMRP. *Journal of experimental neuroscience*, 2015, vol. 9
- **KALRA, V.** ; **SETH, R.** ; **SAPRA, S.** Autism - experiences in a tertiary care hospital. *The Indian Journal of Pediatrics*, 2005, vol. 72 (3), 227-230
- **DAHLHAUS R**. Of Men and Mice: Modeling the Fragile X Syndrome. *frontiers in Molecular NeuroScience*, 2018
- **BAKKER et al.** The Dutch-Belgian Fragile X Consortium (1994) Fmr1 knockout mice: A model to study fragile X mental retardation. *Cell*, vol. 78 (1), 23-33
- **YAN, QJ** ; **ASAFO-ADJEI, PK** ; **ARNOLD, HM** ; **BROWN, RE** ; **BAUCHWITZ, RP**. A phenotypic and molecu-lar characterization of the fmr1-tm1Cgr Fragile X mouse. *Genes, Brain and Behavior*, 2004, vol. 3 (6), 337-359
- **MIENTJES, EJ et al.** The generation of a conditional Fmr1 knockout mouse model to study Fmrp function in vivo. *Neurobiology of Disease*, 2006, vol. 21 (3), 549-555
- **LIU XS** ; **WU H** ; **KRZISCH M** ; **WU X** ; **GRAEF J** ; **MUFFAT J** ; **HNISZ D** ; **LI CH** ; **YUAN B** ; **XU C**. Rescue of fragile X syndrome neurons by DNA methylation editing of the FMR1 gene. *Cell*, 22 February 2018, vol. 172 (5), 979-92
- **MINES MA** ; **YUSKAITIS CJ** ; **KING MK** ; **BEUREL E** ; **JOPE RS**. GSK3 influences social preference and anxiety-related behaviors during social interaction in a mouse model of fragile X syndrome and autism. *PLoS One*, 16 March 2010, vol. 5 (3), e9706
- **SØRENSEN EM** ; **BERTELSEN F** ; **WEIKOP P et al.** Hyperactivity and lack of social discrimination in the adolescent Fmr1 knockout mouse. *Behav Pharmacol.*, 2015, vol. 26 (8), 733-740
- **BERNARDET M** ; **CRUSIO WE**. Fmr1 KO mice as a possible model of autistic features. *ScientificWorldJournal.*, 20 September 2006, vol. 6, 1164-1176
- **ARSENAULT J** ; **GHOLIZADEH S** ; **NIIBORI Y et al.** FMRP Expression Levels in Mouse Central Nervous System Neurons Determine Behavioral Phenotype. *Hum Gene Ther.*, 2016, vol. 27 (12), 982-996
- **DÖLEN G** ; **BEAR MF**. Fragile x syndrome and autism: from disease model to therapeutic targets. *J Neurodev Disord.*, 2009, vol. 1 (2), 133-140
- **HODGES SL** ; **NOLAN SO** ; **TAUBE JH** ; **LUGO JN**. Adult Fmr1 knockout mice present with deficiencies in hippocampal interleukin-6 and tumor necrosis factor-$\alpha$ expression. *Neuroreport*, 2017, vol. 28 (18), 1246-1249
- **DOMANSKI, A.P.F.** ; **BOOKER, S.A.** ; **WYLLIE, D.J.A. et al.** Cellular and synaptic phenotypes lead to disrupted information processing in Fmr1-KO mouse layer 4 barrel cortex. *Nat Commun*, 2019, vol. 10, 4814, https://doi.org/10.1038/s41467-019-12736-y
- **ARBAB, T.** ; **PENNARTZ, C.M.A.** ; **BATTAGLIA, F.P.** Impaired hippocampal representation of place in the Fmr1-knockout mouse model of fragile X syndrome. *Sci Rep*, 2018, vol. 8, 8889, https://doi.org/10.1038/s41598-018-26853-z

- **FILONOVA, IRINA**. Ube3a Role in Synaptic Plasticity and Neurodevelopmental Disorders.The Lessons from Angelman Syndrome.. *Graduate Theses and Dissertations*, 2014, https://scholarcommons.usf.edu/etd/5015
- **COUVERT, P.** ; **BIENVENU, T.** ; **AQUAVIVA, C.** ; **POIRIER, K.** ; **MORAINE, C.** ; **GENDROT, C.** ; **VERLOES, A.** ; **ANDRÈS, C.** ; **LE FEVRE, A.C.** ; **SOUVILLE, I.** MECP2 is highly mutated in X-linked mental retardation. *Human Molecular Genetics*, 2001, vol. 10 (9), 941-946
- **KHATRI, N.** ; **MAN, H.Y.** The autism and Angelman syndrome protein Ube3A/E6AP: The gene, E3 ligase ubiquitination targets and neurobiological functions. *Frontiers in Molecular Neuroscience*, 2019, vol. 12, 109